(19) Europäisches Patentamt
European Patent Office
Office européen des brevets

(11) EP 2 532 689 A1

(12) # EUROPÄISCHE PATENTANMELDUNG

(43) Veröffentlichungstag:
**12.12.2012 Patentblatt 2012/50**

(21) Anmeldenummer: **11168919.6**

(22) Anmeldetag: **07.06.2011**

(51) Int Cl.:
***C08F 220/06*** (2006.01) ***C08F 220/18*** (2006.01)
***A61K 8/81*** (2006.01) ***C08F 265/06*** (2006.01)
***C08F 2/00*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen (DE)**

(72) Erfinder: **Die Erfindernennung liegt noch nicht vor**

(74) Vertreter: **Reinhardt, Jürgen et al**
**BASF Personal Care and Nutrition GmbH**
**Postfach 13 01 64**
**40551 Düsseldorf (DE)**

(54) **Herstellung von Polyacrylaten durch Emulsionspolymerisation**

(57) Die vorliegende Erfindung betrifft ein Verfahren zur Herstellung von Carboxylgruppen enthaltenden Polymeren durch Emulsionspolymerisation und die Verwendung der nach diesem Verfahren erhältlichen Polymere beispielsweise als Verdicker. Die Erfindung betrifft außerdem durch das Verfahren erhältliche Verdicker-Dispersionen und deren Verwendung zur Rheologiemodifizierung von Papier-Streichmassen, Textildruckpasten, Arzneimitteln, kosmetischen Zubereitungen, Waschmitteln, Reinigungsmitteln oder Nahrungsmitteln.

EP 2 532 689 A1

Printed by Jouve, 75001 PARIS (FR)

## Beschreibung

Die vorliegende Erfindung betrifft ein Verfahren zur Herstellung von Carboxylgruppen enthaltenden Polymeren durch Emulsionspolymerisation und die Verwendung der nach diesem Verfahren erhältlichen Polymere beispielsweise als Verdicker. Die Erfindung betrifft außerdem durch das Verfahren erhältliche Verdicker-Dispersionen und deren Verwendung zur Rheologiemodifizierung von Papier-Streichmassen, Textildruckpasten, Arzneimitteln, kosmetischen Zubereitungen, Waschmitteln, Reinigungsmitteln oder Nahrungsmitteln.

EP 13836 offenbart Emulsionscopolymere, die (i) 20 bis 69,5 Gew.-% (Meth)acrylsäure, (ii) 0,5 bis 25 Gew.-% eines Monomers der Formel $CH_2=C(R)-C(O)-O-(CH_2CH_2O)_n-R^0$, worin R für H oder $CH_3$ steht, n wenigstens 2 ist und $R^0$ für $C_8$-$C_{30}$-Alkyl steht, und (iii) wenigstens 30 Gew.-% eines $C_1$-$C_4$-Alkyl(meth)acrylats enthalten. Nach Neutralisation mit Alkali dienen diese Copolymere als Verdicker für Anstrichmittel, Waschmittel und dergleichen.

WO 99/65958 beschreibt alkalilösliche Verdicker, die das Umsetzungsprodukt einer ungesättigten Carbonsäure, eines monoethylenisch ungesättigten Monomers und eines hydrophoben, alkoxylierten Makromonomers umfassen. Das monoethylenisch ungesättigte Monomer umfasst eine Methylgruppe; vorzugsweise handelt es sich um Methylacrylat. Diese Polymere sollen bereits bei pH 4,5 bis 6,0 löslich werden und sich daher für kosmetische Erzeugnisse eignen.

WO 2006/016035 betrifft die Verwendung eines wasserlöslichen Acrylpolymers als Verdickungsmittel in pigmentierten wässrigen Zubereitungen. Das Acrylpolymer besteht aus einem ethylenisch ungesättigten Monomer mit Carboxylfunktion, einem ethylenisch ungesättigten nichtionischen Monomer und einem ethylenisch ungesättigten oxyalkylierten Monomer, das mit einer hydrophoben nicht-aromatischen verzweigten Kette mit 10 bis 24 Kohlenstoffatomen terminiert ist.

WO 2009/062994 betrifft ein Verfahren zur Herstellung einer wässrigen Verdicker-Dispersion aus einer Monomerzusammensetzung, die umfasst:

a) wenigstens eine ethylenisch ungesättigten Carbonsäure, und
b) wenigstens ein ethylenisch ungesättigtes hydrophobes Monomer, wobei man

(i) aus 10 bis 80 Gew.-%, vorzugsweise 40 bis 70 Gew.-%, der Monomerzusammensetzung eine zumindest teilweise polymerisierte Voremulsion herstellt und
(ii) die Restmenge der Monomerzusammensetzung vollständig zu der zumindest teilweise polymerisierten Voremulsion zugibt und eine radikalische Polymerisation initiiert.

Bei der Herstellung von Polymeren mit vergleichsweise großen Mengen an Carboxylgruppen auf dem Wege der herkömmlichen Emulsionspolymerisation kommt es häufig zur Bildung von Koagulat oder Wandbelag im Reaktionsraum, wodurch der Herstellungsprozess in der Regel deutlich erschwert wird.

Die Bildung von feinteiligem Koagulat, auch als Mikrokoagulat oder Stippen bezeichnet, führt unter anderem zur Verstopfung der bei der Aufarbeitung der Dispersionen verwendeten Filter. Stippen sind in der flüssigen Dispersion mit blossem Auge nicht zu erkennen können durch herkömmliche Filtration aber in der Regel nicht abgetrennt werden. Sie beinträchtigen die Weiterverarbeitung der Dispersionen, wie beispielsweise die Herstellung kosmetischer Zubereitungen.

Eine Aufgabe der vorliegenden Erfindung war es daher, ein Verfahren bereitzustellen, das die vorgenannten Nachteile nicht aufweist.

Eine weitere Aufgabe der vorliegenden Erfindung war es, Polymere mit rheologiemodifizierenden Eigenschaften bereitzustellen, die in wässrigem Milieu bei pH>7, bevorzugt pH>8 mit einer Konzentration von wenigstens 1 g pro Liter, bevorzugt wenigstens 10 g pro Liter für das menschliche Auge klar und ohne sichtbare Trübung löslich sind.

Eine weitere Aufgabe der vorliegenden Erfindung war es, Polymere mit rheologiemodifizierenden Eigenschaften bereitzustellen, die die Zubereitung klarer Gele erlauben.

Diese Aufgaben werden gelöst durch ein Verfahren zur Herstellung von Polymeren enthaltend in einpolymerisierter Form

a) M1a+M2a = 30 bis 70 Gew.-% eines Esters der (Meth)Acrylsäure mit einem $C_1$-$C_4$-Alkohol,
b) M1b+M2b = 30 bis 70 Gew.-% (Meth)Acrylsäure,
c) M1c+M2c = 0,1 bis 20 Gew.-% mit wenigstens einem $C_8$-$C_{30}$-Rest substituierte Monomere,
d) M1d+M2d = 0 bis 20 Gew.-% von a) bis c) verschiedene Monomere, durch Emulsionspolymerisation mit einer ersten Polymerisationsstufe und einer zweiten Polymerisationsstufe, dadurch gekennzeichnet, dass
$M1b/M1a \geq 0{,}4$ und
$M1b/M1a < M2b/M2a$ und

$$\sum_{i=a}^{d} M1i \leq 40 \text{ Gew.-\%, wobei}$$

M1i die jeweiligen Gewichtsanteile der Monomere a) bis d) sind, die während der ersten Polymerisationsstufe im Reaktionsraum vorliegen und M2i die jeweiligen Gewichtsanteile der Monomere a) bis d) sind, die während der zweiten Polymerisationsstufe zugegeben werden und die Summe aller Gewichtsanteile

$$\sum_{i=a}^{d} M1i + \sum_{i=a}^{d} M2i = 100 \text{ Gew.-\% ist.}$$

Monomere a)

Als Monomere a) werden Ester der (Meth)Acrylsäure mit einem $C_1$-$C_4$-Alkohol eingesetzt. Das $C_1$-$C_4$-Alkyl-(Meth)acrylat ist bevorzugt ausgewählt aus Methyl(meth)acrylat, Ethyl(meth)acrylat, n-Propyl(meth)acrylat, iso-Propyl(meth) acrylat, n-Butyl(Meth)acrylat, iso-Butyl(meth)acrylat, sec-Butyl(meth)acrylat, tert.-Butyl(meth)acrylat und beliebigen Mischungen daraus.
Besonders bevorzugt sind Methyl(meth)acrylat und Ethyl(meth)acrylat, ganz besonders bevorzugt Ethylacrylat.
Bevorzugt besteht a) zu wenigstens 50 Gew.-%, besonders bevorzugt zu wenigstens 80 Gew.-% und insbesondere zu wenigstens 90 Gew.-% aus Ethylacrylat. In einer Ausführungsform der Erfindung umfasst oder ist a) Ethylacrylat.
Der Gewichtsanteil der Monomere a), der in der ersten Polymerisationsstufe eingesetzt wird, bezogen auf die Gesamtmenge aller während des erfindungsgemäßen Verfahrens zu polymerisierenden Monomere, ist durch M1a gegeben.
Der Gewichtsanteil der Monomere a), der in der zweiten Polymerisationsstufe eingesetzt wird, bezogen auf die Gesamtmenge aller während des erfindungsgemäßen Verfahrens zu polymerisierenden Monomere, ist durch M2a gegeben.
Der gesamte Gewichtsanteil der Monomere a), bezogen auf die Gesamtmenge aller während des erfindungsgemäßen Verfahrens zu polymerisierenden Monomere, ist demnach durch M1a+M2a gegeben und liegt im Bereich M1a+M2a = 30-70 Gew.-%, bevorzugt 30-60 Gew.-%, weiter bevorzugt 40-50 Gew.-%.

Monomere b)

Als Monomer b) wird (Meth)Acrylsäure eingesetzt. Der Begriff "(Meth)Acrylsäure" umfasst vorliegend Acrylsäure, Methacrylsäure sowie Mischungen daraus. Der Begriff (Meth)acrylsäure umfasst dabei jeweils sowohl die neutrale als auch die neutralisierte, anionische Form der (Meth)acrylsäure.
Bevorzugt besteht b) zu wenigstens 50 Gew.-%, besonders bevorzugt zu wenigstens 80 Gew.-% und insbesondere zu wenigstens 90 Gew.-% aus Methacrylsäure. In einer Ausführungsform der Erfindung umfasst oder ist b) Methacrylsäure.
Der Gewichtsanteil der Monomere b), der in der ersten Polymerisationsstufe eingesetzt wird, bezogen auf die Gesamtmenge aller während des erfindungsgemäßen Verfahrens zu polymerisierenden Monomere, ist durch M1b gegeben.
Der Gewichtsanteil der Monomere b), der in der zweiten Polymerisationsstufe eingesetzt wird, bezogen auf die Gesamtmenge aller während des erfindungsgemäßen Verfahrens zu polymerisierenden Monomere, ist durch M2b gegeben.
Der gesamte Gewichtsanteil der Monomere b), bezogen auf die Gesamtmenge aller während des erfindungsgemäßen Verfahrens zu polymerisierenden Monomere, ist demnach durch M1b+M2b gegeben und liegt im Bereich M1b+M2b = 30-70 Gew.-%, bevorzugt 30-60 Gew.-%, weiter bevorzugt 40-50 Gew.-%.

c) mit wenigstens einem $C_8$-$C_{30}$-Rest substituierte Monomere

Die erfindungsgemäß hergestellten Polymere umfassen in einpolymerisierter Form 0,1 bis 20 Gew.-%, bevorzugt 0,1 bis 10 Gew.-%, besonders bevorzugt 0,5 bis 7,5 Gew.-% mit wenigstens einem $C_8$-$C_{30}$-Rest substituierte Monomere c).
Die Monomere c) sind bevorzugt ausgewählt aus Monomeren der allgemeinen Formeln c1) und c2)

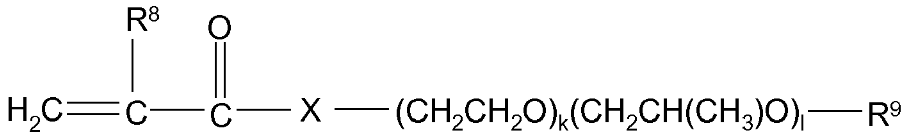

(c1)

$$H_2C{=}CH-CH_2-O-(CH_2CH_2O)_k(CH_2CH(CH_3)O)_l-R^9$$

(c2)

wobei

k und l unabhängig voneinander ganze Zahlen im Bereich von 0 bis 1000 sind und die Summe k+l mindestens gleich 5 ist,
$R^8$ Wasserstoff oder or $C_1$-$C_4$-Alkyl, bevorzugt Methyl, ist
$R^9$ $C_8$-$C_{30}$-Alkyl, $C_8$-$C_{30}$-Alkenyl oder $C_8$-$C_{30}$ Alkylaryl, ist und
X 0 oder $NR^{10}$ ist, wobei $R^{10}$ ausgewählt ist aus H, Alkyl, Alkenyl, Cycloalkyl, Heterocycloalkyl, Aryl oder Hetaryl.

In einer bevorzugten Ausführungsform der Erfindung sind die Monomere c) ausgewählt aus $C_8$-$C_{30}$-Alkylsubstituierten Monomeren.

Unmittelbar an X in c1) bzw. an O in c2) schließt entweder wenigstens ein Ethylenoxidrest (EO) oder wenigstens ein Propylenoxidrest (PO) an. Bevorzugt schließt unmittelbar an X und O wenigstens ein EO an.

In einer Ausführungsform der Erfindung sind die Monomere c1) Ester der (Meth)acrylsäure, also $R^8$ in Formel (c1) ist H oder bevorzugt $CH_3$, mit alkoxylierten Alkoholen.
Geeignete alkoxylierte Alkohole sind beispielsweise die alkoxylierten

- linearen Alkohole aus natürlichen Quellen oder aus der Ziegler-Aufbaureaktion von Ethylen in Gegenwart von Aluminiumalkyl-Katalysatoren. Beispiele für geeignete lineare Alkohole sind lineare $C_8$-$C_{30}$-Alkohole, insbesondere $C_{12}$-$C_{30}$-Alkohole. Als besonders bevorzugte Alkohole seien genannt: n-Dodecanol, n-Tetradecanol, n- Hexadecanol, n-Octadecanol, n-Eicosanol, n-Docosanol, n-Tetracosanol, n-Hexacosanol, n-Octacosanol, und/oder n-Triacontanol, sowie Gemische der vorstehend genannten Alkohole, beispielsweise NAFOL® Typen wie NAFOL® 22+ (Sasol).
- Oxoalkohole wir bespielsweise Isooctanol, Isononanol, Isodecanol, Isoundecanol, Isotridecanol (beispielsweise Exxal®-Typen 7, 8, 9, 10, 11, 13).
- Alkohole, die in 2-Stellung verzweigt sind; hierbei handelt es sich um die dem Fachmann bekannten Guerbetalkohole, die durch Dimerisierung von primären Alkoholen über die so genannte Guerbetreaktion zugänglich sind. Als besonders bevorzugte Alkohole seien hier genannt: Isofol®12 (Sasol), Rilanit®G16 (Cognis).

- Alkohole, die durch die Friedel-Crafts-Alkylierung mit oligomerisierten Oleflnen erhalten werden und die dann neben einem gesaettigten Kohlenwasserstoffrest einen aromatischen Ring enthalten. Als besonders bevorzugte Alkohole seien hier genannt: i-Octylphenol und i-Nonylphenol.
- Alkohole der allgemeinen Formel (4) der EP 761780 A2, S.4

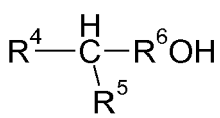

oder Alkohole der allgemeinen Formel (5) der EP 761780 A2, S.4

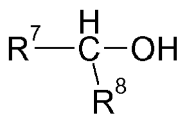

,

wobei

- $R^4$, $R^5$, $R^7$ und $R^8$ unabhängig voneinander die in EP 761780 A2, S.4, Zeilen 45 bis 58 beschriebene Bedeutung haben; bevorzugt sind $R^4$, $R^5$, $R^7$ und $R^8$ unabhängig voneinander Alkylreste mit wenigstens 4 Kohlenstoffatomen und die Gesamtzahl der Kohlenstoffatome der Alkohole beträgt höchstens 30,
- $R^6$ ein Alkylenrest wie beispielsweise $-CH_2-$, $-CH_2-CH_2-$, $-CH_2-CH(CH_3)-$; Beispielsweise sei hier 2-Decyl-1-tetradecanol als geeigneter Alkohol genannt.

In einer Ausführungsform ist wenigstens ein c) ein (Meth)acrylsäureester eines Gemisches ethoxylierter $C_8$-$C_{30}$-, bevorzugt $C_{12}$-$C_{30}$-, insbesondere $C_{16}$-$C_{22}$-Fettalkohole.

In einer Ausführungsform ist wenigstens ein c) ein (Meth)acrylsäureester eines Gemisches ethoxylierter $C_8$-$C_{30}$-, bevorzugt $C_{12}$-$C_{30}$-, insbesondere $C_{16}$-$C_{22}$-Fettalkohole, wobei die ethoxylierten Alkohole jeweils 20 bis 30 EO-Reste umfassen.

In einer Ausführungsform ist wenigstens ein c) ein (Meth)acrylsäureester eines Gemisches ethoxylierter $C_{12}$-$C_{18}$-Fettalkohole, wobei die ethoxylierten Alkohole jeweils 10 bis 30 EO-Reste umfassen.

In einer Ausführungsform der Erfindung umfasst oder ist c) ein Methacrylsäureester eines mit 25 Mol Ethylenoxid ethoxylierten $C_{16}$-$C_{18}$-Fettalkoholes (auch als "$C_{16-18}$-Alkyl-PEG1100 - methacrylate" bezeichnet).

Kommerziell erhältlich sind solche $C_{16-18}$-Alkyl-PEG1100 - methacrylate beispielsweise als Plex®6877-O (25 Gew.-%ige Zubereitung in Methylmethacrylat) oder Lutencryl®250 (50 Gew.-%ige Lösung in Methacrylsäure) oder VISIOMER® C18 PEG 1105 MA.

In einer Ausführungsform der Erfindung umfasst oder ist c) ein Methacrylsäureester eines mit 25 Mol Ethylenoxid ethoxylierten $C_{18}$-$C_{22}$-Fettalkoholes (auch als "Beheneth-25 methacrylate" bezeichnet, CAS-Nr. 115047-92-2), kommerziell beispielsweise als Sipomer® BEM erhältlich.

In einer weiteren Ausführungsform der Erfindung umfasst c) wenigstens zwei unterschiedliche (Meth)acrylsäureester ethoxylierter $C_8$-$C_{30}$-, bevorzugt ethoxylierter $C_{12}$-$C_{30}$-, insbesondere ethoxylierter $C_{16}$-$C_{22}$-Fettalkohole.

Gegenstand der vorliegenden Erfindung ist auch ein erfindungsgemäßes Verfahren, dadurch gekennzeichnet, dass c) 0,1 bis 20 Gew.-% eines Esters der (Meth)Acrylsäure mit wenigstens einem Alkohol der allgemeinen Formel (I)

$$H\text{-}(O\text{-}CHR\text{-}CH_2)_x\text{-}O\text{-}C_yH_{2y+1} \qquad (I)$$

wobei x eine ganze Zahl von 10 bis 50 ist, y eine ganze Zahl von 12 bis 30 ist und R ausgewählt ist aus H und $CH_3$, in einpolymerisierter Form enthalten sind.

Bevorzugt ist das erfindungsgemäße Verfahren dadurch gekennzeichnet, dass c) wenigstens zwei verschiedene Monomere der Formel (I) umfasst.

Besonders bevorzugt ist das erfindungsgemäße Verfahren weiterhin dadurch gekennzeichnet, dass y eine ganze Zahl von 16 bis 22 ist.

Weitere geeignete Monomere c) sind Verbindungen der allgemeinen Formel c1), wobei $R^8$ gleich H oder bevorzugt Methyl, X gleich O, k und l gleichzeitig Null und $R^9$ $C_8$-$C_{20}$-Alkylaryl oder bevorzugt $C_8$-$C_{20}$- Alkyl.

Beispiele für solche Monomere a3) sind n-Octyl(meth)acrylat, 1,1,3,3-Tetramethylbutyl(meth)acrylat, 2-Ethylhexyl(meth) acrylat, n-Nonyl(meth)acrylat, n-Decyl(meth)acrylat, Isodecyl(meth)acrylat, n-Undecyl(meth)acrylat, Isounde-cyl(meth) acrylat, Dodecyl(meth)acrylat, Tridecyl(meth)acrylat, Myristyl(meth)acrylat, Pentadecyl(meth)acrylat, Palmityl(meth) acrylat, Heptadecyl(meth)acrylat, Stearyl(meth)acrylat, Lauryl(meth)acrylat, Arrachinyl(meth)acrylat, Behenyl(meth) acrylat, Lignocerenyl(meth)acrylat, Cerotinyl(meth)acrylat, Melissinyl(meth)acrylat, Palmitolei- nyl(meth)acrylat, Oleyl (meth)acrylat, Linolyl(meth)acrylat, Linolenyl(meth)acrylat und Mischungen davon.

Der Gewichtsanteil der Monomere c), der in der ersten Polymerisationsstufe eingesetzt wird, bezogen auf die Gesamtmenge aller während des erfindungsgemäßen Verfahrens zu polymerisierenden Monomere, ist durch M1c gegeben.

Der Gewichtsanteil der Monomere c), der in der zweiten Polymerisationsstufe eingesetzt wird, bezogen auf die Gesamtmenge aller während des erfindungsgemäßen Verfahrens zu polymerisierenden Monomere, ist durch M2c gegeben.

Der gesamte Gewichtsanteil der Monomere c), bezogen auf die Gesamtmenge aller während des erfindungsgemäßen Verfahrens zu polymerisierenden Monomere, ist demnach durch M1c+M2c gegeben und liegt im Bereich M1c+M2c = 0,1-20 Gew.-%, bevorzugt 0,1-15 Gew.-%, besonders bevorzugt 0,5-7,5 Gew.-%.

M1c beträgt bevorzugt höchstens 0,1*(M1c+M2c), weiter bevorzugt höchstens 0,01*(M1c+M2c) und insbesondere 0.

Bevorzugt wird also der überwiegende oder der vollständige Gewichtsanteil von c) während der zweiten Polymerisationsstufe einpolymerisiert.

Monomere d)

Die erfindungsgemäß erhältlichen Polymere umfassen in einpolymerisierter Form gegebenenfalls 0 bis 20 Gew.-% weitere, von a) bis c) verschiedene Monomere d).
Als Monomere d) geeignet sind grundsätzlich alle kosmetisch akzeptablen, radikalisch polymerisierbaren Verbindungen. Insbesondere sind als Monomere d) auch vernetztende Verbindungen mit mehr als einer radikalisch polymerisierbaren Doppelbindung geeignet.
Der Gewichtsanteil der Monomere d), der in der ersten Polymerisationsstufe eingesetzt wird, bezogen auf die Gesamtmenge aller während des erfindungsgemäßen Verfahrens zu polymerisierenden Monomere, ist durch M1d gegeben.
Der Gewichtsanteil der Monomere d), der in der zweiten Polymerisationsstufe eingesetzt wird, bezogen auf die Gesamtmenge aller während des erfindungsgemäßen Verfahrens zu polymerisierenden Monomere, ist durch M2d gegeben.
Der gesamte Gewichtsanteil der Monomere d), bezogen auf die Gesamtmenge aller während des erfindungsgemäßen Verfahrens zu polymerisierenden Monomere, ist demnach durch M1d+M2d gegeben und liegt im Bereich M1d+M2d = 0-20 Gew.-%, bevorzugt 0-10 Gew.-%, besonders bevorzugt 0,1-7,5 Gew.-%.
Bei dem erfindungsgemäßen Verfahren handelt es sich bevorzugt um eine Emulsionspolymerisation. Die Emulsionspolymerisation an sich ist ein seit langem im Stand der Technik bekanntes Verfahren zur Herstellung von Polymeren in wässriger Phase und bedarf keiner weiteren Erläuterung an dieser Stelle.
Das erfindungsgemäße Verfahren umfasst zwei Polymerisationsstufen, wobei die zweite Polymerisationsstufe zeitlich nach der ersten Polymerisationsstufe beginnt.
Die erste Polymerisationsstufe umfasst

1.1) die Bereitstellung der sogenannten Vorlage enthaltend die Monomer-Teilmengen M1a, M1b, M1c, und M1d, Wasser, gegebenenfalls weitere Verbindungen, bevorzugt wenigstens ein oberflächenaktives Mittel,
1.2) die Erwärmung der aus 1.1) erhaltenen Mischung auf eine Temperatur im Bereich von 30 bis 120°C, bevorzugt 40 bis 90°C,
1.3) die Zugabe eines Polymerisationsinitiators.

Die zweite Polymerisationsstufe beginnt nach Schritt 1.3) der ersten Polymerisationsstufe und umfasst

2.1) die Zugabe der Monomer-Teilmengen M2a, M2b, M2c und M2d zur aus 1.3) erhaltenen Reaktionsmischung und
2.2) Nachbehandlung der aus 2.1) erhaltenen Reaktionsmischung wie beispielsweise Verringerung der Restmonomere, Filtration, wenigstens teilweise Neutralisation.

Die in Schritt 2.1) erfolgende Zugabe der Monomer-Teilmengen M2a, M2b, M2c und M2d zur aus 1.3) erhaltenen Reaktionsmischung kann erfindungsgemäß auf verschiedene Weise erfolgen:

- die Monomere können zeitlich und räumlich unabhängig voneinander als getrennte Zuläufe zugegeben werden. Die einzelnen Zuläufe können gleichzeitig oder nacheinander begonnen und beendet werden;
- ein Teil der Monomere kann gemeinsam als Mischung, der übrige Teil in getrennten Zuläufen gleichzeitig oder nacheinander zugegeben werden;
- die Monomere können alle gemeinsam als Mischung in einem Zulauf zugegeben werden.

Eine bevorzugte Ausführungsform der Erfindung ist das erfindungsgemäße Verfahren, dadurch gekennzeichnet, dass eine Mischung umfassend alle Teilmengen M2i, also $\sum_{i=a}^{d} M2i$ gemeinsam und gleichzeitig in einem Zulauf zugegeben wird. Besonders bevorzugt ist diese Mischung eine wässrige Emulsion enthaltend alle Monomer-Teilmengen M2i und wenigstens ein oberflächenaktives Mittel.
Erfindungsgemäß gilt für die Mengenverhältnisse der Monomeren a) und b): M1b/M1a ≥ 0,4 und M1b/M1a < M2b/M2a.
Weiterhin sind M1a, M2a, M1b und M2b jeweils größer Null. Bevorzugt ist auch M2c größer Null.
Bevorzugt ist M1 b/M1 a ≥ 0,5, weiter bevorzugt ≥ 0,6.
Erfindungsgemäß sind während der ersten Polymerisationsstufe höchstens 40 Gew.-%, bevorzugt höchstens 25 Gew.-% und besonders bevorzugt höchstens 12 Gew.-% aller zu polymerisierenden Monomere im Reaktionsraum

vorhanden, d.h. $\sum_{i=a}^{d} M1i \leq 40$ Gew.-%, bevorzugt ≤ 25 Gew.-%, besonders bevorzugt ≤ 12 Gew.-%.

Eine Ausführungsform der Erfindung ist ein Verfahren zur Herstellung von Polymeren enthaltend in einpolymerisierter Form

a) M1a+M2a = 30 bis 50 Gew.-% eines Esters der (Meth)Acrylsäure mit einem $C_1$-$C_4$-Alkohol,
b) M1 b+M2b = 30 bis 50 Gew.-% (Meth)Acrylsäure,
c) M1c+M2c = 0,1 bis 20 Gew.-% mit wenigstens einem $C_8$-$C_{30}$-Rest substituierte Monomere,

Mld+M2d = 0 bis 20 Gew.-% von a) bis c) verschiedene Monomere,
durch Emulsionspolymerisation mit einer ersten Polymerisationsstufe und einer zweiten Polymerisationsstufe, dadurch gekennzeichnet, dass
M1b/M1a ≥ 0,6 und
M1b/M1a < M2b/M2a und

$$\sum_{i=a}^{d} M1i \leq 12 \text{ Gew.-\%},$$

wobei
M1i die jeweiligen Gewichtsanteile der Monomere a) bis d) sind, die während der ersten Polymerisationsstufe im Reaktionsraum vorliegen und
M2i die jeweiligen Gewichtsanteile der Monomere a) bis d) sind, die während der zweiten Polymerisationsstufe zugegeben werden und die Summe aller Gewichtsanteile

$$\sum_{i=a}^{d} M1i + \sum_{i=a}^{d} M2i = 100 \text{ Gew.-\% ist.}$$

Das erfindungsgemäße Verfahren wird bevorzugt bei pH<7, weiter bevorzugt bei pH<5 und insbesondere bei pH<3 durchgeführt.

Die Polymerisation wird bevorzugt durch einen thermisch aktivierbaren Initiator oder einen Redoxinitiator initiiert. Die Verwendung eines thermisch aktivierbaren Initiators ist besonders bevorzugt. Geeignete thermisch aktivierbare radikalische Initiatoren sind vor allem solche des Peroxy- und Azotyps. Hierzu zählen unter anderem Wasserstoffperoxid, Peressigsäure, t-Butylhydroperoxid, Di-t-butylperoxid, Dibenzoylperoxid, Benzoylhydroperoxid, 2,4-Dichlorbenzoylperoxid, 2,5-Dimethyl-2,5-bis(hydroperoxy)hexan, Perbenzoesäure, t-Butylperoxypivalat, t-Butylperacetat, Dilauroyl peroxid, Dicapryloylperoxid, Distearoylperoxid, Dibenzoylperoxid, Diisopropylperoxydicarbonat, Didecylperoxydicarbonat, Dieicosylperoxydicarbonat, Di-t-butylperbenzoate, Azobisisobutyronitril, 2,2'-Azobis-2,4-dimethylvaleronitril, Ammoniumpersulfat, Kaliumpersulfat, Natriumpersulfat und Natriumperphosphat.
Erfindungsgemäß bevorzugt werden Persulfate (Peroxodisulfate) verwendet, insbesondere Natriumpersulfat.
Bei der Durchführung der Emulsionspolymerisation wird der Initiator in ausreichender Menge verwendet, um die Polymerisationsreaktion zu initiieren. Der Initiator wird üblicherweise in einer Menge von etwa 0,01 bis 3 Gew.-%, bezogen auf das Gesamtgewicht der zu polymerisierenden Monomere, verwendet. Die Initiatormenge beträgt vorzugsweise etwa 0,05 bis 2 Gew.-% und insbesondere 0,1 bis 1 Gew.-%, bezogen auf das Gesamtgewicht der zu polymerisierenden Monomere.
In einer Ausführungsform der Erfindung wird die ausreichende Menge des Initiators während der ersten Polymerisationsstufe zur Vorlage gegeben.

Das erfindungsgemäße Verfahren erfolgt in der Regel in Gegenwart eines anionischen und/oder nichtionischen Emulgators.
Typische Emulgatoren sind anionische Emulgatoren wie z. B. Natriumlaurylsulfat, Natriumtridecylethersulfate, Dioctylsulfosuccinat Natriumsalz und Natriumsalze von Alkylarylpolyethersulfonaten und nichtionische Emulgatoren wie z. B. Alkylarylpolyetheralkohole und Ethylenoxid-Propylenoxid-Copolymere.
Bevorzugte Emulgatoren weisen die allgemeine Formel R-O-($CH_2$-CHR'-O)$_n$-X auf, worin R für $C_6$-$C_{30}$-Alkyl, R' für Wasserstoff oder Methyl, X für Wasserstoff oder $SO_3M$, M für Wasserstoff oder ein Alkalimetall, und n für eine Zahl von

2 bis 100 stehen.

Die erfindungsgemäß erhaltene Polymerdispersion kann einer chemischen Desodorierung unterzogen werden. Bei der chemischen Desodorierung wird nach Ende der eigentlichen Emulsionspolymerisation weiterer Initiator, z.B. ein Redoxinitiator zugesetzt. Zur chemischen Desodorierung geeignete Redoxinitiatoren umfassen als oxidierende Komponente beispielsweise wenigstens ein organisches Peroxid und/oder Hydroperoxid wie Wasserstoffperoxid, tert.-Butylperoxid, Cumolhydroperoxid, Pinanhydroperoxid, Diisopropylphenylhydroperoxid, Dibenzoylperoxid, Dilauroylperoxid und Diacetylperoxid und als reduzierend wirkende Komponente beispielsweise Alkalimetallsulfite, Ascorbinsäure, Acetonbisulfit-Addukt und/oder ein Alkalimetallsalz der Hydroxymethansulfinsäure, wobei Eisen(II)-salze, vorzugsweise in komplexierter Form, als Katalysator zugesetzt werden können.

Die erfindungsgemäß erhältlichen Polymerdispersionen weisen in der Regel einen Feststoffgehalt von 20 bis 60 Gew.-%, insbesondere etwa 30 bis 40 Gew.-%, auf.

In unneutralisierter Form weisen die erfindungsgemäß erhältlichen Polymerdispersionen eine relativ niedrige Viskosität auf. Sie sind daher leicht handhabbar und können problemlos dosiert oder umgepumpt werden. Durch Neutralisation, z. B. auf einen pH von mehr als 5, vorzugsweise mehr als 6, insbesondere 8 bis 10, wird das Copolymer löslich und die Viskosität des wässrigen Mediums steigt stark an. Dabei werden vorzugsweise mehr als 50 Mol-% der Säuregruppen neutralisiert. Geeignete Neutralisierungsmittel sind z. B. Natriumhydroxid, Kaliumhydroxid, Ammoniumhydroxid, Amine, wie Triethylamin, Triethanolamin, Monoethanolamin, und andere alkalische Materialien.

Die nicht neutralisierten erfindungsgemäßen Dispersionen haben den besonderen Vorteil, dass sie sehr gut filtrierbar sind.

Bevorzugt sind nicht neutralisierte erfindungsgemäße Dispersionen, dadurch gekennzeichnet, dass 120 Liter der erfindungsgemäßen Polymerdispersion mit einem Feststoffgehalt von 30 Gew-% bei 25 °C und 1,5 bar Vordruck eine Filterkombination von zwei Filtern in höchstens 5 Minuten, vorzugsweise höchstens 3 Minuten durchströmt, wobei

- der erste Filter ein Nylon-Monofilament-Gewebe-Filter mit einer Filterfeinheit von 125 µm, einem Durchmesser von 180 mm, einer Länge von 430 mm und einer Filterfläche von ca. 0.25 $m^2$ ist (Produktbezeichnung NMO-125-P01S-60M, Fa. Eaton),
- der zweite Filter ein Polypropylen-Nadelfilz-Filter mit einer Filterfeinheit von 25 µm, einem Durchmesser von 180 mm, einer Länge von 430 mm und einer Filterfläche von 0.25 $m^2$ ist (Produktbezeichnung PO-25-P01S-60L, Fa. Eaton) und
- die beiden Filter durch einen Schlauch von 2 m Länge und einem Leitungsquerschnitt von 20 mm verbunden sind.

Besonders bevorzugt sind nicht neutralisierte erfindungsgemäße Dispersionen, dadurch gekennzeichnet, dass 120 Liter der erfindungsgemäßen Polymerdispersion mit einem Feststoffgehalt von 30 Gew-% bei 25 °C und 1,5 bar Vordruck eine Filterkombination von zwei Filtern in höchstens 5 Minuten, vorzugsweise höchstens 3 Minuten durchströmt, wobei

- der erste Filter ein Nylon-Monofilament-Gewebe-Filter mit einer Filterfeinheit von ≤ 125 µm, einem Durchmesser von ≤ 180 mm, einer Länge von ≤ 430 mm und einer Filterfläche von ≤ ca. 0.25 $m^2$ ist (Produktbezeichnung NMO-125-P01 S-60M, Fa. Eaton),
- der zweite Filter ein Polypropylen-Nadelfilz-Filter mit einer Filterfeinheit von ≤ 25 µm, einem Durchmesser von ≤ 180 mm, einer Länge von ≤ 430 mm und einer Filterfläche von ≤ ca. 0.25 $m^2$ ist (Produktbezeichnung PO-25-P01S-60L, Fa. Eaton) und

die beiden Filter durch einen Schlauch von ≥ 2 m Länge und einem Leitungsquerschnitt von ≤ 20 mm verbunden sind.

Die besonders gute Filtrierbarkeit der erfindungsgemäßen Dispersionen führt dazu, dass große Mengen von bis zu 1200 Liter der nicht neutralisierten Dispersionen mit einem Feststoffgehalt von 30 Gew.-% unter vorgenannten Bedingungen filtriert werden können, ohne dass ein Filterwechsel erforderlich wäre.

Kosmetische Zubereitungen

Die Erfindung betrifft weiterhin kosmetische Zubereitungen, die die durch das erfindungsgemäße Verfahren erhältlichen Polymere umfassen.

Solche erfindungsgemäßen kosmetische Zubereitungen sind beispielsweise ausgewählt aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern.

Weitere erfindungsgemäße kosmetische Zubereitungen sind hautkosmetische Zubereitungen, insbesondere solche zur Pflege der Haut. Diese liegen insbesondere als W/O-oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weiterhin eignen sich die erfindungsgemäß erhältlichen Polymere als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäß erhältlichen Polymere verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere bevorzugte erfindungsgemäße Zubereitungen sind Wasch-, Dusch- und Badepräparate, die die erfindungsgemäß erhältlichen Polymere enthalten.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden.

Die erfindungsgemäßen kosmetischen Zubereitungen können als wässrige oder wässrig-alkoholische Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Die erfindungsgemäßen kosmetischen Zubereitungen enthalten bevorzugt wenigstens ein erfindungsgemäß erhältlichen Polymere, wenigstens einen kosmetisch akzeptablen Träger und wenigstens einen davon verschiedenen Bestandteil der ausgewählt ist unter kosmetisch aktiven Wirkstoffen, Emulgatoren, Tensiden, Konservierungsmitteln, Parfümölen, weiteren Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Erfindungsgemäß bevorzugte Haarpflegemittel sind ausgewählt aus Vorbehandlungsmitteln, Haarspülungen, Haarconditionern, Haarbalsamen, leave-on-Haarkuren, rinseoff-Haarkuren, Haarwässern, Pomaden, Frisiercremes, Frisierlotionen, Frisiergelen, Spitzenfluids, Hot-Oil-Treatments und Schaumkuren.

Bevorzugt werden die erfindungsgemäß erhältlichen Polymere als rheologiemodifizierende Filmbildner, Haarfestiger und Konditioniermittel zur Herstellung von kosmetischen, bevorzugt haarkosmetischen Zubereitungen verwendet.

Ein weiterer Gegenstand der Erfindung sind also kosmetische, insbesondere haarkosmetische Zubereitungen enthaltend die erfindungsgemäß erhältlichen Polymere.

Bevorzugte haarkosmetische Zubereitungen sind Haarreinigungsmittel, Shampoos, Haarpflegemittel, Haarfärbezubereitungen und Haarfestiger, darunter insbesondere Haarfestigergele.

Die erfindungsgemäß erhältlichen Polymere wirken insbesondere als filmbildende und/oder konditionierende Rheologiemodifizierungsmittel. Sie eignen sich somit speziell für Haarfestiger als "verdickende Festiger" oder "festigende Verdicker" und in Haarpflegemitteln als "konditionierende Verdicker".

Prinzipiell können die erfindungsgemäß erhältlichen Polymere bei ihrer Verwendung in mehrphasigen Zubereitungen wie beispielsweise O/W und W/O, sowohl in der Wasserphase als auch in der Ölphase eingesetzt werden. Im Allgemeinen enthalten heterogenphasige flüssig/flüssig-Zubereitungen die erfindungsgemäß erhältlichen Polymere im Wesentlichen in der Wasserphase.

Ein weiterer Gegenstand der Erfindung sind haarkosmetische Mittel enthaltend

A) wenigstens ein erfindungsgemäß erhältliches Polymer,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff.

Die erfindungsgemäß erhältlichen Polymere in den haarkosmetischen Mitteln auch als haarfestigende Komponente eingesetzt werden, so dass der Einsatz weiterer Festigerpolymere nur in verringerter Menge erforderlich ist oder sogar ganz überflüssig sein kann.

Die erfindungsgemäß erhältlichen Polymere zeichnen sich vorteilhafterweise auch durch konditionierende Eigenschaften aus und können die sensorischen Eigenschaften des Haars verbessern, z. B. ihm Geschmeidigkeit und Glanz verleihen.

Die haarkosmetischen Mittel enthalten die erfindungsgemäß erhältlichen Polymere vorzugsweise in einem

Anteil von etwa 0,1 bis 10 Gew.-%, besonders bevorzugt 0,2 bis 6 Gew.-%, insbesondere 0,3 bis 3 Gew.-%, bezogen auf das Gesamtgewicht des Mittels.

Beispiele für geeignete Haarpolymere B) und deren bevorzugte Mengen sind in WO 2007/010035, S.68, Z.32 bis S.70, Z.22 ausführlich beschrieben. Auf diese Textstelle wird hiermit in vollem Umfang Bezug genommen.

Vorzugsweise weisen die Zubereitungen eine Trägerkomponente C) auf, die ausgewählt ist unter Wasser, hydrophilen Komponenten, hydrophoben Komponenten und Mischungen davon.

Geeignete Trägerkomponenten C) sind in WO 2007/010035, S.70, Z.28 bis S.71, Z.37 ausführlich beschrieben. Auf diese Textstelle wird hiermit in vollem Umfang Bezug genommen.

Zusätzlich können die erfindungsgemäßen Mittel als Komponente D) wenigstens einen weiteren, von A) und B) verschiedenen kosmetischen Wirk- oder Hilfsstoff enthalten. Geeignete Komponenten D) sind in WO 2007/010035, S.72, Z.2 bis S.72, Z.13 ausführlich beschrieben. Auf diese Textstelle wird hiermit in vollem Umfang Bezug genommen.

Die erfindungsgemäß erhältlichen Polymere können gemeinsam mit bekannten Verdickern verwendet werden. Geeignete Verdicker sind in WO 2007/010035, S.72, Z.15 bis S.72, Z.24 ausführlich beschrieben. Auf diese Textstelle wird hiermit in vollem Umfang Bezug genommen.

Konditionierungsmittel

Als Konditionierungsmittel für die erfindungsgemäßen kosmetischen Zubereitungen werden bevorzugt diejenigen Konditionierungsmittel gewählt, die auf Seite 34, Zeile 24 bis Seite 37, Zeile 10 der WO 2006/106140 beschrieben sind, worauf hiermit Bezug genommen wird.

Verdicker

Für Gele, Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird. Geeignete weitere Verdickungsmittel für die erfindungsgemäßen kosmetischen Zubereitungen sind beispielsweise auch auf Seite 37, Zeile 12 bis Seite 38, Zeile 8 der WO 2006/106140 beschrieben, worauf hiermit Bezug genommen wird.

Konservierungsmittel

Geeignete Konservierungsmittel für die erfindungsgemäßen kosmetischen Zubereitungen sind beispielsweise auf Seite 38, Zeile 10 bis Seite 39, Zeile 18 der WO 2006/106140 beschrieben, worauf hiermit Bezug genommen wird.

UV- Lichtschutzfilter

Geeignete UV- Lichtschutzfilter für die erfindungsgemäßen kosmetischen Zubereitungen sind beispielsweise auf Seite 39, Zeile 20 bis Seite 41 Zeile 10 der WO 2006/106140 beschrieben, worauf hiermit Bezug genommen wird.

Antioxidantien

Geeignete Antioxidantien für die erfindungsgemäßen kosmetischen Zubereitungen sind beispielsweise auf Seite 41, Zeile 12 bis Seite 42 Zeile 33 der WO 2006/106140 beschrieben, worauf hiermit Bezug genommen wird.

Dispergiermittel

Wenn in den erfindungsgemäßen Zubereitungen unlösliche Wirkstoffe, z.B. Antischuppenwirkstoffe oder Siliconöle, dispergiert und auf Dauer in Schwebe gehalten werden sollen, werden bevorzugt Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt.

Die Zubereitungen können weitere in der Kosmetik übliche Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile wie Alkohole, Polyole, Polymere, organische Säuren zur pH-Wert-Einstellung, Schaumstabilisatoren, Elektrolyte, organische Lösemittel oder Silikonderivate enthalten.
Hinsichtlich der genannten, dem Fachmann bekannten weiteren Inhaltsstoffe für die Zubereitungen sei auf "Kosmetik und Hygiene von Kopf bis Fuß", Hrsg. W. Umbach, 3. Auflage, Wiley-VCH, 2004, S.123-128 verwiesen, worauf hiermit Bezug genommen wird.

Die erfindungsgemäßen Zubereitungen wie Haarsprays, Gele, Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glycerylolead-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat. Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.
Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

Wirkstoffe

Vorteilhafte Wirkstoffe für die erfindungsgemäßen kosmetischen Zubereitungen sind beispielsweise auf Seite 44, Zeile 24 bis Seite 49 Zeile 39 der WO 2006/106140 beschrieben, worauf hiermit Bezug genommen wird.

UV-Lichtschutzmittel

Die erfindungsgemäßen Zubereitungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird.,

Perlglanzwachse

Geeignete Perlglanzwachse für die erfindungsgemäßen kosmetischen Zubereitungen sind beispielsweise auf Seite 50, Zeile 1 bis Zeile 16 der WO 2006/106140 beschrieben, worauf hiermit Bezug genommen wird.

Emulgatoren

Die erfindungsgemäßen kosmetischen Zubereitungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben, worauf hiermit Bezug genommen wird.

Parfümöle

Sollen den erfindungsgemäßen kosmetischen Zubereitungen Parfumöle zugesetzt werden, so sind geeignete Parfümöle beispielsweise auf Seite 53, Zeile 10 bis Seite 54, Zeile 3 der WO 2006/106140 beschrieben, worauf hiermit Bezug genommen wird.

Pigmente

Gegebenenfalls enthalten die erfindungsgemäßen kosmetischen Zubereitungen weiterhin Pigmente. Geeignete Pigmente für die erfindungsgemäßen Zubereitungen sind beispielsweise auf Seite 54, Zeile 5 bis Seite 55, Zeile 19 der WO 2006/106140 beschrieben, worauf hiermit Bezug genommen wird.

Nanopartikel

Gegebenenfalls enthalten die erfindungsgemäßen Zubereitungen wasserunlösliche Nanopartikel, also Teilchen mit einer Teilchengröße im Bereich von 1 bis 200, bevorzugt von 5 bis 100 nm. Bevorzugte Nanopartikel sind Nanopartikel von Metalloxiden, insbesondere von Zinkoxid und/oder Titandioxid.

Polymere

Die erfindungsgemäßen kosmetischen Zubereitungen enthalten in einer bevorzugten Ausführungsform außer den erfindungsgemäß erhältlichen Polymeren noch weitere Polymere. Geeignete weitere Polymere sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

Die erfindungsgemäß erhältlichen Polymere sind auch als rheologiemodifizierende Filmbildner in Haargelen, insbesondere sogenannten Stylinggelen geeignet.

Eine bevorzugte Ausführungsform der Erfindung sind haarkosmetische Zubereitungen, insbesondere Haarfestiger und Haargele, die neben den erfindungsgemäß erhältlichen Polymeren in der Kosmetik übliche Gelbildner enthalten.

Solche weiteren, üblichen Gelbildner sind leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Haarwaschmittel

Eine bevorzugte Ausführungsform der Erfindung sind Haarwaschmittel und Shampoos enthaltend die erfindungsgemäß erhältlichen Polymere.

An Shampoos und Haarwaschmittel werden je nach Haarqualität oder Kopfhautproblem gegebenenfalls zusätzliche Anforderungen gestellt.

Bevorzugte erfindungsgemäße Shampoos und Haarwaschmittel enthalten anionische Tenside. Weitere bevorzugte erfindungsgemäße Shampoos und Haarwaschmittel enthalten Kombinationen von anionischen und ampholytischen Tensiden. Weitere bevorzugte erfindungsgemäße Shampoos und Haarwaschmittel enthalten Kombinationen von anionischen und zwitterionischen Tensiden. Weitere bevorzugte erfindungsgemäße Shampoos und kosmetische Reinigungsmittel enthalten Kombinationen von anionischen und nichtionischen Tensiden.

Geeignete Tenside aller Typen sind zuvor unter "Tenside" bereits beschrieben. Bevorzugte anionische Tenside sind Alkylsulfate, Alkylpolyglykolethersulfate und E-thercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen. Besonders bevorzugte anionische Tenside sind die Alkali- oder Ammoniumsalze des Laurylethersulfates mit einem Ethoxylierungsgrad von 2 bis 4 EO-Einheiten.

Ein bevorzugtes zwitterionisches Tensid ist das unter der INCI-Bezeichnung Cocamidopropyl Betaine bekannte Fettsäureamid-Derivat.

Besonders bevorzugte ampholytische Tenside sind das N-Kokosalkylaminopropionat, das Kokosacylaminoethylaminopropionat und das C12-C12-Acylsarcosin.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Darreichung

Die erfindungsgemäßen Zubereitungen können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels. Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Dimethylether, Kohlenwasserstoffe (Propan, Butan, Isobutan) geeignet, die allein oder in Mischung, beispielsweise Mischungen aus Dimethylether und Isobutan oder Dimethylether und Butan, miteinander eingesetzt werden können. Auch Druckluft, Stickstoff, Stickstoffdioxid oder Kohlendioxid oder Gemische aus diesen Substanzen sind vorteilhaft zu verwenden.

Die Herstellung der erfindungsgemäßen Zubereitungen kann in der üblichen Weise durch Mischen der einzelnen Bestandteile erfolgen. Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat

oder Phosphorsäure/Phosphat-Puffergemischen. In einer Ausführungsform der Erfindung liegt der pH-Wert unter 10, z.B. im Bereich von 2-7, insbesondere im Bereich von 3-5.

Bevorzugte Shampooformulierungen enthalten

a) 0,05 bis 10 Gew.-% wenigstens eines erfindungsgemäß erhältlichen Polymers,
b) 25 bis 94,95 Gew.-% Wasser,
c) 5 bis 50 Gew.-% Tenside,
d) 0 bis 5 Gew.-% eines Konditioniermittels,
e) 0 bis 10 Gew.-% weitere kosmetische Bestandteile.

In einer weiteren Ausführungsform können durch den Einsatz der erfindungsgemäß erhältlichen Polymere auch tensidreduzierte Formulierungen mit weniger als 10 Gew.-% Tensid, bezogen auf die Zubereitung, in einer für die Zubereitung ausreichenden Viskosität hergestellt werden.

In den Shampoos und kosmetischen Reinigungsmitteln können alle in Shampoos und kosmetischen Reinigungsmitteln üblicherweise eingesetzten anionischen, neutralen, amphoteren oder kationischen Tenside verwendet werden. Geeignete Tenside wurden vorstehend genannt. Besonders bevorzugt sind Shampoos und kosmetische Reinigungsmittel mit einem Tensidgehalt von mehr als 10 Gew.-%.

In den Shampooformulierungen können zur Erzielung bestimmter Effekte weitere Konditioniermittel eingesetzt werden. Hierzu zählen beispielsweise kationische Polymere mit der INCI-Bezeichnung Polyquaternium, insbesondere Copolymere aus Vinylpyrrolidon/N-Vinylimidazoliumsalzen (Luviquat®FC, Luviquat®HM, Luviquat®MS, Luviquat®Care), Copolymere aus N-Vinylpyrrolidon/Dimethylaminoethylmethacrylat, quaternisiert mit Diethylsulfat (Luviquat®PQ 11), Copolymere aus N-Vinylcaprolactam/N-Vinylpyrrolidon/N-Vinylimidazoliumsalzen (Luviquat®Hold); kationische Cellulosederivate (Polyquaternium-4 und -10), Acrylamidcopolymere (Polyquaternium-7).
Vorteilhafte Konditionierungsmittel stellen beispielsweise die nach INCI als Polyquaternium bezeichneten Verbindungen dar (insbesondere Polyquaternium-1 bis Polyquaternium-87).

Beispiele

Die Erfindung wird nachfolgend durch Beispiele näher beschrieben, ohne sie darauf zu beschränken. Sofern nicht anders angegeben, bedeuten Mengenangaben in "%" Gewichtsprozent.

Beispiel 1

Der Versuchsaufbau bestand aus einem 2-L-Reaktionsgefäß mit Ankerrührer, Rückflußkühler und Zulaufgefäßen.

In der Vorlage wurden 360,0g VE-Wasser, 0,6g Natriumdodecylsulfat, 6,0g Methacrylsäure und 9,0g Ethylacrylat unter Rühren (120Upm) auf 80°C aufgeheizt.
Beim Erreichen von 80°C wurden 24,0g einer 2,5%igen wässrigen Natriumperoxodisulfatlösung für den Reaktionsstart schnell zugegeben.
5min nach der Zugabe der Natriumperoxodisulfatlösung wurde eine gerührte Emulsion bestehend aus

| | |
|---|---|
| 210,0g | VE-Wasser |
| 1,65g | Natriumdodecylsulfat |
| 7,2g | Sorbitantristearat-20 EO (Tween®80) |
| 30,0g | Methacrylsäureester eines ethoxylierten C16 - C18-Fettalkoholgemisches (Visiomer®C18 PEG-1105-MA 60%ig) |
| 8,6g | Methacrylsäureester eines ethoxylierten C18 - C22-Fettalkoholgemisches (Sipomer® BEM 52,5%ig) |
| 136,5g | Methacrylsäure |
| 126,0g | Ethylacrylat |

in 1,5 h in das Reaktionsgefäß zudosiert. Nach Zulaufende rührte der Versuch noch 2h bei 80°C nach. Anschließend wurde der Versuch auf Raumtemperatur abgekühlt. Beim Erreichen von 40°C während des Abkühlprozesses erfolgte die Zugabe von 0,9g einer 1 %igen wässrigen Wasserstoffperoxidlösung. 5 min nach dieser Zugabe wurden

30,0g wässrige 0,25%ige L(+)-Ascorbinsäure in 30 min zudosiert.

Der pH-Wert der 30,3 Gew.-%igen Dispersion (Feststoffgehalt 30,3 Gew.-%) betrug ca. 2,8.

Die Dispersion wurde dann über einen 120μm Filter abfiltriert und dann über einen Filter mit Maschenweite 25 μm abfiltriert. Die Filtrierbarkeit war sehr gut.

Im Filter und am Rührer klebend ließen sich 0,7 g Koagulat nachweisen (gemessen als feuchtes Koagulat nach Abpressen mit einem trockenen Papiertuch).

Durch tropfenweise Zugabe (unter Rühren) von Triethanolamin zu der vorher mit Wasser verdünnten milchigen Dispersion (Feststoffgehalt nach der Verdünnung 5 Gew.-%) entstand ab einem pH-Wert von 7 eine wasserklare Polymerlösung.

Beispiel 2

Der Versuchsaufbau bestand aus einem 2-L-Reaktionsgefäß mit Ankerrührer, Rückflußkühler und Zulaufgefäßen.

In der Vorlage wurden 720,0g voll entsalztes Wasser (VE-Wasser), 1,2g Natriumdodecylsulfat, 12,0g Methacrylsäure und 18,0g Ethylacrylat unter Rühren (120Upm) auf 80°C aufgeheizt.

Beim Erreichen von 80°C wurden 48,0g einer 2,5%igen wässrigen Natriumperoxodisulfatlösung für den Reaktionsstart schnell zugegeben.

5 min nach der Zugabe der Natriumperoxodisulfatlösung wurde eine gerührte Emulsion bestehend aus

| | |
|---|---|
| 420,0g | VE-Wasser |
| 3,30g | Natriumdodecylsulfat |
| 14,4g | Sorbitantristearat-20 EO (Tween®80) |
| 75,0g | Methacrylsäureester eines ethoxylierten C16 - C18-Fettalkoholgemisches (Visiomer®C18 PEG-1105-MA 60%ig) |
| 273,0g | Methacrylsäure |
| 237,0g | Ethylacrylat |

in 1,5h in das Reaktionsgefäß zudosiert.

Nach Zulaufende rührte der Versuch noch 2h bei 80°C nach. Anschließend wurde der Versuch auf Raumtemperatur abgekühlt. Beim Erreichen von 40°C während des Abkühlprozesses erfolgte die Zugabe von 1,8g einer 1 %igen wässrigen Wasserstoffperoxidlösung. 5min nach dieser Zugabe wurden 60,0g wässrige 0,25%ige L(+)-Ascorbinsäure in 30min zudosiert.

Der pH-Wert der 30,9 % Gew.-%-igen Dispersion betrug ca. 2,8. Die Dispersion wurde zunächst über einen Filter mit Maschenweite 120 μm und dann über einen Filter mit Maschenweite 25 μm abfiltriert. Die Filtrierbarkeit war sehr gut.

Im 120 μm Filter und am Rührer (klebend) ließen sich 0,9 g Koagulat nachweisen (gemessen als feuchtes Koagulat nach Abpressen mit einem trockenen Papiertuch).

Durch tropfenweise Zugabe (unter Rühren) von Triethanolamin zu der vorher mit Wasser verdünnten milchigen Dispersion (Feststoffgehalt nach der Verdünnung 5 Gew.%) entstand ab einem pH-Wert von 7 eine wasserklare Polymerlösung.

Beispiel 3

Der Versuchsaufbau bestand aus einem 2-L-Reaktionsgefäß mit Ankerrührer, Rückflußkühler und Zulaufgefäßen.

In der Vorlage wurden 360,0g VE-Wasser, 0,6g Natriumdodecylsulfat, 6,0g Methacrylsäure und 9,0g Ethylacrylat unter Rühren (120Upm) auf 80°C aufgeheizt.

Beim Erreichen von 80°C wurden 24,0g einer 2,5%igen wässrigen Natriumperoxodisulfatlösung für den Reaktionsstart schnell zugegeben.

5min nach der Zugabe der Natriumperoxodisulfatlösung wurde eine gerührte Emulsion bestehend aus

| | |
|---|---|
| 210,0g | VE-Wasser |
| 1,65g | Natriumdodecylsulfat |
| 7,2g | Sorbitantristearat-20 EO (Tween®80) |

(fortgesetzt)

| | |
|---|---|
| 25,0g | Methacrylsäureester eines ethoxylierten C16 - C18-Fettalkoholgemisches (Visiomer®C18 PEG-1105-MA 60%ig) |
| 139,0g | Methacrylsäure |
| 126,0g | Ethylacrylat |

in 1,5 h in das Reaktionsgefäß zudosiert. Nach Zulaufende rührte der Versuch noch 2h bei 80°C nach. Anschließend wurde der Versuch auf Raumtemperatur abgekühlt. Beim Erreichen von 40°C während des Abkühlprozesses erfolgte die Zugabe von 0,9g einer 1%igen wässrigen Wasserstoffperoxidlösung. 5 min nach dieser Zugabe wurden 30,0g wässrige 0,25%ige L(+)-Ascorbinsäure in 30 min zudosiert.

Der pH-Wert der 30,3 %igen Dispersion betrug ca. 2,8.

Die Dispersion wurde dann über einen 120 µm Filter abfiltriert. Die Filtrierbarkeit war sehr gut.

Im Filter und am Rührer (klebend) ließen sich 0,5 g Koagulat nachweisen (gemessen als feuchtes Koagulat nach Abpressen mit einem trockenen Papiertuch).

Durch tropfenweise Zugabe (unter Rühren) von Triethanolamin zu der vorher mit Wasser verdünnten milchigen Dispersion (Feststoffgehalt nach der Verdünnung 5 Gew.%) entstand ab einem pH-Wert von 7 eine wasserklare Polymerlösung.

Vergleichsbeispiel 1

Der Versuchsaufbau bestand aus einem 2-L-Reaktionsgefäß mit Ankerrührer, Rückflußkühler und Zulaufgefäßen.

In der Vorlage wurden 720,0g VE-Wasser, 1,2g Natriumdodecylsulfat, 20,0g Methacrylsäure und 10,0g Ethylacrylat unter Rühren (120Upm) auf 80°C aufgeheizt.

Beim Erreichen von 80°C wurden 48,0g einer 2,5%igen wässrigen Natriumperoxodisulfatlösung für den Reaktionsstart schnell zugegeben.

5 min nach der Zugabe der Natriumperoxodisulfatlösung wurde eine gerührte Emulsion bestehend aus

| | |
|---|---|
| 420,0g | VE-Wasser |
| 3,30g | Natriumdodecylsulfat |
| 14,4g | Sorbitantristearat-20 EO (Tween®80) |
| 75,0g | Methacrylsäureester eines ethoxylierten C16 - C18-Fettalkoholgemisches (Visiomer®C18 PEG-1105-MA 60%ig) |
| 273,0g | Methacrylsäure |
| 237,0g | Ethylacrylat |

in 1,5h in das Reaktionsgefäß zudosiert. Nach Zulaufende rührte der Versuch noch 2h bei 80°C nach. Anschließend wurde der Versuch auf Raumtemperatur abgekühlt. Beim Erreichen von 40°C während des Abkühlprozesses erfolgte die Zugabe von 1,8g einer 1%igen wässrigen Wasserstoffperoxidlösung. 5min nach dieser Zugabe wurden 60,0g wässrige 0,25%ige L(+)-Ascorbinsäure in 30min zudosiert.

Der pH-Wert der 30,9 %igen Dispersion betrug ca. 2,8.

Die Dispersion wurde dann über einen 120µm Filter abfiltriert. Die Filtration verlief langsamer als bei den Beispielen 1 und 2.

Im 120 µm Filter und am Rührer (klebend) ließen sich 3 g Koagulat nachweisen (gemessen als feuchtes Koagulat nach Abpressen mit einem trockenen Papiertuch).

Durch tropfenweise Zugabe (unter Rühren) von Triethanolamin zu der vorher mit Wasser verdünnten milchigen Dispersion (Feststoffgehalt nach der Verdünnung 5 Gew.%) entsteht ab einem pH-Wert von ca. 7 eine wasserklare Polymerlösung.

Vergleichsbeispiel 2

Der Versuchsaufbau bestand aus einem 2-L-Reaktionsgefäß mit Ankerrührer, Rückflußkühler und Zulaufgefäßen.

In der Vorlage wurden 720,0g VE-Wasser, 1,2g Natriumdodecylsulfat, 5,0g Methacrylsäure und 25,0g Ethylacrylat unter Rühren (120Upm) auf 80°C aufgeheizt.

Beim Erreichen von 80°C wurden 48,0g einer 2,5%igen wässrigen Natriumperoxodisulfatlösung für den Reaktionsstart schnell zugegeben.
5min nach der Zugabe der Natriumperoxodisulfatlösung wurde eine gerührte Emulsion bestehend aus

| | |
|---|---|
| 420,0g | VE-Wasser |
| 3,30g | Natriumdodecylsulfat |
| 14,4g | Sorbitantristearat-20 EO (Tween®80) |
| 75,0g | Methacrylsäureester eines ethoxylierten C16 - C18-Fettalkoholgemisches (Visiomer®C18 PEG-1105-MA 60%ig) |
| 273,0g | Methacrylsäure |
| 237,0g | Ethylacrylat |

in 1,5 Stunden in das Reaktionsgefäß zudosiert. Nach Zulaufende rührte der Versuch noch 2 Stunden bei 80°C nach. Anschließend wurde der Versuch auf Raumtemperatur abgekühlt. Beim Erreichen von 40°C während des Abkühlprozesses erfolgte die Zugabe von 1,8g einer 1 %igen wässrigen Wasserstoffperoxidlösung. 5 min nach dieser Zugabe wurden 60,0g wässrige 0,25%ige L(+)-Ascorbinsäure in 30min zudosiert.

Der pH-Wert der 30,9 %igen Dispersion betrug ca. 2,8. Die Dispersion wurde dann über einen 120μm Filter abfiltriert. Die Filtrierbarkeit war sehr gut.
Im Filter und am Rührer (klebend) ließen sich 0,8 g Koagulat nachweisen (gemessen als feuchtes Koagulat nach Abpressen mit einem trockenen Papiertuch).

Im Gegensatz zu den Polymeren der erfindungsgemäßen Beispiele 1 und 2 und des Vergleichsbeispiels 1 ließ sich die saure Dispersion durch Zugabe von Triethanolamin nicht klar auflösen, sie blieb trüb.

Zusammenfassung der Ergebnisse:

| | B1 | B2 | B3 | V1 | V2 |
|---|---|---|---|---|---|
| M1b/M1a | 0.67 | 0.67 | 0.67 | 2 | 0.2 |
| M2b/M2a | 1.08 | 1.15 | 1.10 | 1.15 | 1.15 |
| M1b/M1a < M2b/M2a | erfüllt | erfüllt | erfüllt | Nicht erfüllt | erfüllt |
| Koagulat | Wenig | Sehr wenig | Sehr wenig | Viel | Sehr wenig |
| Filtrierbarkeit 120μm | | | Sehr gut | Schlecht | Sehr gut |
| Filtrierbarkeit 120μm + 25 μm | Sehr gut | Sehr gut | | | |
| Klarheit | Gut | Gut | Gut | Gut | mangelhaft |
| Methacrylsäu re Restmo nomer | < 10 ppm | < 10 ppm | < 10 ppm | < 10 ppm | < 10 ppm |

Beschreibung der Prüfungen

Bestimmung der Viskosität

Die Viskosität wurde mit einem Viskosimeter des Typs Brookfield DV-II+Pro bei 20°C gemessen. Alle Messungen wurden bei einer Umdrehungsgeschwindigkeit von 20 Upm durchgeführt. Für die Messungen wurden je nach Viskositätsbereich, Messspindeln der Grössenordnung No.6 oder No.7 verwendet.
Die Viskositätsmessung wurde am fertig formulierten Gel im 250-ml-Glasgebinde durchgeführt und erfolgte 24 Stunden nach der Gelherstellung.

Bestimmung der Klarheit

Die Beurteilung der Klarheit eines Gels erfolgt ausschließlich durch das menschliche Auge. Jeweils zwei Personen beurteilen unter Tageslichtbedingungen das Aussehen der jeweiligen Standard-Gelformulierung in einem 250-ml-Glasgebinde.

Durchführung des Biegetests

Vorbereitung der Proben

Das jeweils zu prüfende Polymer wurde in eine Gelformulierung eingearbeitet. 50g des erhaltenen Gels wurden mit voll entsalztem Wasser (VE-Wasser) auf 220g aufgefüllt und gelöst. Die gewogenen, trockenen Haarsträhnen (ca. 3g, 24cm Länge) wurden in die verdünnte Gelmasse getaucht. Durch dreimaliges Eintauchen, Herausnehmen und Abstreifen wurde eine gleichmässige Verteilung der Gellösung auf dem Haar sichergestellt.
Die überschüssige Masse wurde zwischen Daumen und Zeigefinger abgestreift, die Haarsträhne anschließend durch Ausdrücken zwischen Filterpapier auf eine Gewichtszunahme von 1g - 1,4g (bezogen auf das Ausgangsgewicht der Haarsträhne, je nach Viskosität der Masse) eingestellt.
Danach wurden die Strähnen von Hand so geformt, dass sie einen etwa runden Querschnitt erhielten. Bei 20°C und 65% relativer Feuchte wurden die Strähnen über Nacht im Klimaraum getrocknet.

Prüfung der Biegefestigkeit

Die Prüfungen wurden jeweils in einem Klimaraum bei 20°C und 65% relativer Feuchte mittels eines Zug/Druck-Prüfgerätes (Maschinen -Typ: TT 2803 E6) durchgeführt.
Die Haarsträhne wurde symmetrisch auf zwei zylindrische Rollen (Durchmesser 4 mm, Abstand = 90mm) der Probenaufnahme gelegt. Genau in der Mitte wurde nun von oben mit einem abgerundeten Stempel die Strähne ca. 40 mm bis zum Brechen des Gelfilmes durchgebogen. Die dafür erforderliche Kraft wurde mit einer Kraftmesszelle (50 N) gemessen und in Newton angegeben. Für einen repräsentativen Biegetestwert wurden 7 Haarsträhnen vermessen und der Mittelwert gebildet.

Bestimmung der Gelstruktur

Das zu prüfende Gel wurde mittels Flachspatel auf eine Glasplatte aufgetragen und gleichmässig verteilt. Von mindestens 2 Personen wurde die aufgetragene Gelstruktur beurteilt. Beurteilungskriterien waren z.B. glatt, narbig, klumpig, rau und klare Filmbildung.

pH-Messung

Der pH-Wert des Geles wurde mit einer Glaselektrode (Einstabmesskette) bei Raumtemperatur und im unverdünnten Gel bestimmt. Verwendetes Messgerät: pH-Meter der Fa.Knick, Typ: Portamess.

Salzstabilität (schiefe Ebene / Rampe)

Herstellung der Lösung

Für die Untersuchung der Gele auf der schiefen Ebene wurde die Zusammensetzung von menschlichem Schweiß aus folgenden Substanzen nachgestellt:

20,0 g Kochsalz
17,5 g Ammoniumchlorid
5,0 g Essigsäure
15,0 g Milchsäure

Die Substanzen wurden in 1,0 Liter VE- Wasser gelöst und mittels Natronlauge auf einen pH- Wert von 4,8 eingestellt. Vor jeder Verwendung wurde der pH- Wert der Lösung überprüft und gegebenenfalls auf 4,8 korrigiert.

Aufbau und Durchführung:

Auf einem 6 x 10 cm großen Filterpapier wurden Start- und Ziellinie markiert. Der Abstand zwischen beiden Linien betrug 5 cm und entsprach der Lauflänge, die das Gel zurücklegte. Als schiefe Ebene wurde eine Glasplatte genutzt, die in einen verschließbaren Glasbehälter hineingestellt wurde (ca. 60 ° bis 65 °). Auf diese Glasplatte wurde das markierte Filterpapier aufgelegt und mit 4 mL der künstlichen Schweiß- Lösung bzw. VE- Wasser getränkt. Es wurde darauf geachtet, dass das Filterpapier gleichmäßig benetzt war. Etwa 0,3 g des jeweils zu untersuchenden Gels wurden auf das mit Lösung getränkte Papier aufgetragen. Mittels einer Stoppuhr wurde die Zeit gemessen, die das Gel benötigte, um 5 cm auf der Ebene zu fließen. In Abbildung 1 ist der Versuchsaufbau schematisch dargestellt.

Um den Basiswert eines einzelnen Gels zu bestimmen, wurde der Versuch zuerst mit VE- Wasser als Fließmittel durchgeführt. Wenn das Gel nach 15 Minuten die Zielposition noch nicht erreicht hatte, wurde der Versuch abgebrochen. Die Zeit und die zurückgelegte Strecke wurden dokumentiert.
Im Anschluss wurde der Versuch mit künstl. Schweiß als Fließmittel wiederholt. Die Zeit, die das Gel benötigte, um die 5 cm zurückzulegen, wurde notiert. Jeder Versuch (mit VE- Wasser und künstlichem Schweiß) wurde viermal durchgeführt, wobei der Mittelwert und die relative Standardabweichung berechnet wurden.

Haargele

Die im Folgenden dargestellten erfindungsgemäßen Haargele wurden wie folgt hergestellt:

Zunächst wurden die Komponenten der Phase A solubilisiert. Dann wurde Phase B angesetzt und gelöst, anschliessend in Phase A gegeben und unter Rühren eine gemeinsame Lösung hergestellt. Schließlich wurde Phase C in Phase A+B gegeben und wurde bis zur Homogenität gerührt.
Die angegebenen Mengen sind in Gew.-% sofern nicht ausdrücklich anders beschrieben.
Dispersion 1 ist die filtierte Polymerdispersion des erfindungsgemäßen Beispiels 1.

Anstelle von Dispersion 1 kann in allen folgenden Rezepturen jede beliebige erfindungsgemäße Polymerdispersion eingesetzt werden, insbesondere auch die Polymerdispersionen der erfindungsgemäßen Beispiele 2 und 3.

Haargel 1

| Ingredient | INCI (Hersteller) |
|---|---|
| Phase A | |
| 0,40 Cremophor® CO40 | PEG-40 |
| Hydrogenated Castor Oil | |
| 0,10 Parfümöl | |
| 81,09 Wasser dest. | Aqua dem. |
| Phase B | |
| 15,00 Luviskol® K90 (3% Polymergehalt) | PVP |
| 2,57 Dispersion 1 (0,8% WS) | |
| 0,50 Euxyl® PE 9010 | Phenoxyethanol Ethylhexyl Glycerin |
| Phase C | |
| 0,34 AMP | 2-Amino-2-Methyl-propanol |
| Prüfdaten: Viskosität | 37100 mPas (Brookfield® RVD VII+) |

Haargel 2

| Ingredient | INCI (Hersteller) |
|---|---|
| Phase A | |
| 0,40 Cremophor® CO40 | PEG-40Hydrogenated Castor Oil |
| 0,10 Parfümöl | |
| 85,45 Wasser dest. | Aqua dem. |

(fortgesetzt)

| Ingredient | INCI (Hersteller) |
|---|---|
| Phase B | |
| 10,00 Luviskol® VA64W (5% Polymergehalt) | PVP/VA |
| 3,21 Dispersion 1 (1,0% Polymergehalt) | |
| 0,50 Euxyl® PE 9010 | Phenoxyethanol Ethylhexyl Glycerin |
| Phase C | |
| 0,34 AMP | 2-Amino-2Methyl-propanol |
| Prüfdaten: | |
| Viskosität | 38900 mPas (Brookfield RVD VII+) |
| pH-Wert | 7,10 |

Haargel 3

| Ingredient | INCI (Hersteller) |
|---|---|
| Phase A | |
| 0,40 Cremophor® CO40 | PEG-40Hydrogenated Castor Oil |
| 0,10 Parfümöl | |
| 80,45 Wasser dest. | Aqua dem. |
| Phase B | |
| 15,00 Luviset® Clear (3% Polymergehalt) | VP/Methacrylamide/N-Vinyl Imidazole Polymer |
| 3,21 Dispersion 1 (1,0% Polymergehalt) | |
| 0,50 Euxyl® PE 9010 | Phenoxyethanol Ethylhexyl Glycerin |
| Phase C | |
| 0,34 AMP | 2-Amino-2Methyl-propanol |
| Prüfdaten: | |
| Viskosität | 40000 mPas (Brookfield RVD VII+) |
| pH-Wert | 7,10 |

Haargel 4

| Ingredient | INCI (Hersteller) |
|---|---|
| Phase A | |
| 0,40 Cremophor® CO40 | PEG-40Hydrogenated Castor Oil |
| 0,10 Parfümöl | |
| 85,45 Wasser dest. | Aqua dem. |
| Phase B | |
| 2,50 Luviquat® Supreme (0,5% Polymergehalt) | Polyquaternium-68 |
| 7,50 Luviset® Clear (1,50% Polymergehalt) | VP/Methacrylamide/N-Vinyl Imida zole Polymer |
| 3,21 Dispersion 1 (1,0% Polymergehalt) | |
| 0,50 Euxyl® PE 9010 | Phenoxyethanol Ethylhexyl Glyce- |
| rin | |
| Phase C | |
| 0,34 AMP | 2-Amino-2Methyl-propanol |

(fortgesetzt)

| Ingredient | INCI (Hersteller) |
|---|---|
| Prüfdaten: | |
| Viskosität | 56600 mPas (Brookfield RVD VII+) |
| pH - Wert | 7,20 |
| Biegetest | 200 cN |

Haargel 5

| Ingredient | INCI (Hersteller) | |
|---|---|---|
| Phase A | | |
| 0,40 Cremophor® CO40 | PEG-40 Hydrogenated Castor Oil | |
| 0,10 Parfümöl | | |
| 77,95 Wasser dest. | Aqua dem. | |
| Phase B | | |
| 2,50 Luviquat® Supreme (0,5% Polymergehalt) | | Polyquaternium-68 |
| 15,00 Luviskol® K90 (3,0% Polymergehalt) | | PVP |
| 3,21 Dispersion 1 (1,0% Polymergehalt) | | |
| 0,50 Euxyl® PE 9010 | | Phenoxyethanol Ethylhexyl Glycerin |
| Phase C | | |
| 0,34 AMP | | 2-Amino-2-Methyl-propanol |
| Prüfdaten: | | |
| Viskosität | 68800 mPas (Brookfield RVD | VII+) |
| pH-Wert | 7,12 | |
| Biegetest | 196 cN | |

Haargel 6

| Ingredient | INCI (Hersteller) |
|---|---|
| Phase A | |
| 0,40 Cremophor® CO40 | PEG-40 Hydrogenated Castor Oil |
| 0,10 Parfümöl | |
| 82,95 Wasser dest. | Aqua dem. |
| Phase B | |
| 2,50 Luviquat® Supreme (0,5% Polymergehalt) | Polyquaternium-68 |
| 1,00 Luviskol® VA64 (5,0% Polymergehalt) | PVP/VA |
| 3,21 Dispersion 1 (1,0% Polymergehalt) | |
| 0,50 Euxyl® PE 9010 | Phenoxyethanol Ethylhexyl Glycerin |
| Phase C | |
| 0,34 AMP | Amino-2-Methyl-propanol |
| Prüfdaten: | |
| Viskosität | 41750 mPas (Brookfield RVD VII+) |
| pH-Wert | 6,90 |
| Biegetest | 166 cN |

Haargel 7

| Ingredient | INCI (Hersteller) |
|---|---|
| Phase A | |
| 0,30 Cremophor® CO40 | PEG-40 Hydrogenated Castor Oil |
| 0,10 Parfümöl | |
| 79,84 Wasser dest. | Aqua dem. |
| Phase B | |
| 10,00 Luviset® Clear (2,0% Polymergehalt) | VP/Methacrylamide/N-Vinyl Imidazole Polymer |
| 3,33 Dispersion 1 (1,0% Polymergehalt) | |
| 2,50 Karion®F | Sorbitol |
| 2,50 1,2 Propylenglykol Care | Propylenglykol |
| 0,50 Panthenol 50P | Panthenol |
| 0,10 Niacinamid | Niacinamid (Nutrilo) |
| 0,50 Euxyl®PE 9010 | Phenoxyethanol Ethylhexyl Glycerin |
| Phase C | |
| 0,33 AMP | 2-Amino-2Methyl-propanol |
| Prüfdaten: | |
| Viskosität 45200 mPas (Brookfield RVD VII+) | |
| pH-Wert 7,30 | |
| Biegetest 236 cN | |
| Curl-Retention 89% | |

Haargel 8

| Ingredient | INCI (Hersteller) |
|---|---|
| Phase A | |
| 0,30 Cremophor® CO40 | PEG-40Hydrogenated Castor Oil |
| 0,10 Parfümöl | |
| 74,84 Wasser dest. | Aqua dem. |
| Phase B | |
| 5,00 Luviskol®VA 64W (2,5% Polymergehalt) | PVP/VA Copolymer |
| 10,00 Luviset®Clear (2,0% Polymergehalt) | VP/Methacrylamide/N-Vinyl Imida zole Polymer |
| 3,33 Dispersion 1 (1,0% Polymergehalt) | |
| 2,50 Karion®F | Sorbitol |
| 2,50 1,2 Propylenglykol Care | Propylenglykol |
| 0,50 Panthenol 50P | Panthenol |
| 0,10 Niacinamid | Niacinamid (Nutrilo) |
| 0,50 Euxyl®PE 9010 | Phenoxyethanol Ethylhexyl Glycerin |
| Phase C | |
| 0,33 AMP | 2-Amino-2Methyl-propanol |
| Prüfdaten: | |
| Viskosität | 37800 mPas (Brookfield RVD VII+) |
| pH - Wert | 7,20 |

Vergleich verschiedener Verdicker in Zubereitungen mit PVP (Luviskol®K90)

| | INCI | Verdickeranteil (%) | Viskosität (mPas) | pH-Wert | Klarheit | Biegetest (cN) | Struktur glatt / rauh Note 1-4 | Salzverträglichkeit |
|---|---|---|---|---|---|---|---|---|
| Polymer nach Beispiel 1 | | 1% | 64400 | 6,97 | klar | 175 ±14 | glatt | Ja |
| Aculyn®22 | Acryla tes/ Steareth-20 Methacrylate Copolymer | 1% | 30700 | 7,08 | klar | 150 ±15 | glatt | Ja |
| Aculyn®28 | Acrylates/ Beheneth 25 Methacrylate Copolymer | 1% | 120000 | 7,19 | fast klar | 198 ±15 | rauh | Ja |
| Aculyn®33 | Acrylates Copolymer | 1% | 5920 | 7,00 | trüb | 75 ±6 | glatt | Nein |
| Aculyn®88 | Acrylates Copolymer | 1% | 12380 | 7,08 | klar | 74 ±6 | glatt | Nein |
| Structure® 3001 | Acrylates/Ceteth-20-Itacone Copolymer | 1% | 3560 | 7,13 | klar | 119±7 | glatt | Ja |
| Salcare®SC80 | Steareth-10 Allylether Acrylates Copolymer | 1% | 13840 | 6,93 | klar | 129 ±14 | glatt | Ja |
| Salcare® SC81 | Acrylates Copolymer | 1% | 9300 | 7,14 | trüb | 81 ±14 | glatt | Nein |
| Tinovis® GTC | Acrylates/ Beheneth 25 Methacrylate Copolymer | 1% | 110000 | 7,00 | klar | 171 ±13 | rauh | Ja |
| Carbopol® 980 | Carbomer | 0,5% | 78800 | 7,03 | trüb | 97 ±8 | fast glatt | Nein |

Ergebnis: Nur das erfindungsgemäß erhältliche Polymer ermöglicht in Haargelen die Kombination der wichtigen Anwendungseigenschaften Lösungsviskosität, Klarheit des Gels, hohe Festigerleistung, glatte Gelstruktur und Salzverträglichkeit.

Haarshampoos

Die angegebenen Mengen sind in Gew.-% sofern nicht ausdrücklich anders beschrieben.
Dispersion 1 ist die filtierte Polymerdispersion des erfindungsgemäßen Beispiels 1.

Conditioning Shampoo mit Jaguar® C 13 S und Siliconen

| Ingredient | INCI (Hersteller) |
|---|---|
| Phase A | |
| 0,20 Jaguar®C 13 S | Guar Hydroxypropyltrimonium Chloride |
| 62,00 Wasser dest. | Aqua dem. |
| Phase B | |
| 17,50 Texapon®N 701 (12%) | Sodium laureth Sulfate |
| 5,40 Tego®Betain L7 (1,6%) | Cocoamidopropyl Betaine (Evonik) |
| Phase C | |
| 1,60 Dispersion 1 | |
| q.s. NaOH 20% | Sodium Hydroxide |
| Phase D | |
| 2,20 Dow Corning®Dispersion 1785 (1,3%) | Dimethiconol, TEA-Dodecylbenzenesulfonate |
| 1,10 DowCorning®Emulsion CE-8170 (0,2%) | Amodimethocone, C11-15 Pareth-7, Glycerin, Trideceth-12 |
| 9,10 Euperlan®3000 AM (2%) | Glycol Distearate, Laureth-4, Cocamidopropyl Betaine |
| 0,20 Parfümöl | |
| 0,20 Glydant® LTD. | DMDM Hydantoin |
| Phase E | |
| q.s. Citronensäure | Citric Acid |
| 0,50 Natriumchlorid | Natriumchlorid |

Herstellung:

Die Komponenten der Phase A wurden gelöst. Phase B wurde angesetzt und in Phase A gegeben. Phase C wurde in Phase A+B gegeben und der pH-Wert unter Rühren auf ungefähr 6,7 eingestellt. Phase D wurde in die Mischung A+B+C homogen eingerührt.
Der pH-Wert der erhaltenen Mischung wurde mit Citronensäure auf ungefähr pH 6,0 eingestellt, Natriumchlorid wurde zugegeben und alles homogen gerührt.

| Prüfdaten: | |
|---|---|
| Viskosität | 5300 mPas (Brookfield RVD VII+) |
| pH-Wert | 6,10 |

Conditioning Shampoo mit UCARE ® Polymer 400C und Siliconen

| Ingredient | INCI (Hersteller) |
|---|---|
| Phase A | |
| 0,20 UCARE® Polymer JR 400 | Polyquaternium-10 |
| 62,00 Wasser dest. | Aqua dem. |
| Phase B | |
| 17,50 Texapon®N 701 (12%) | Sodium laureth Sulfate |
| 5,40 Tego®Betain L7 (1,6%) | Cocoamidopropyl Betaine (Evonik) |
| Phase C | |
| 1,60 Dispersion 1 | |
| q.s. NaOH 20% | Sodium Hydroxide |
| Phase D | |
| 2,20 Dow Corning®Dispersion 1785 (1,3%) | Dimethiconol, TEA-Dodecylbenzenesulfonate |
| 1,10 DowCorning®Emulsion CE-8170 (0,2%) | Amodimethocone, C11-15 Pareth-7, Glycerin, Trideceth-12 |
| 9,10 Euperlan®3000 AM (2%) | Glycol Distearate, Laureth-4, Cocamidopropyl Betaine |
| 0,20 Parfümöl | |
| 0,20 Glydant® LTD. | DMDM Hydantoin |
| Phase E | |
| q.s. Citronensäure | Citric Acid |
| 0,50 Natriumchlorid | Natriumchlorid |

Herstellung:

Die Komponenten der Phase A wurden gelöst. Phase B wurde angesetzt und in Phase A gegeben. Phase C wurde in Phase A+B gegeben und der pH-Wert unter Rühren auf ungefähr 6,7 eingestellt. Phase D wurde in die Mischung A+B+C homogen eingerührt.
Der pH-Wert der erhaltenen Mischung wurde mit Citronensäure auf ungefähr pH 6,0 eingestellt, Natriumchlorid wurde zugegeben und alles homogen gerührt.

| Prüfdaten: | |
|---|---|
| Viskosität | 4800 mPas (Brookfield RVD VII+) |
| pH-Wert | 5,95 |

Conditioning Shampoo mit geringerem Tensidanteil

| Ingredient | INCI (Hersteller) |
|---|---|
| Phase A | |
| 3,30 Dispersion 1 | |
| 59,80 Wasser dest. | Aqua dem. |

(fortgesetzt)

| Ingredient | INCI (Hersteller) |
|---|---|
| q.s. NaOH 20% | Sodium Hydroxide |
| Phase B | |
| 0,10 Salcare®CS 60 | Acrylamidopropyltrimonium Chloride |
| 2,90 Wasser dest. | Aqua dem. |
| Phase C | |
| 25,80 Texapon®NSO (7% Tensidanteil) | Sodium Laureth Sulfate |
| 6,80 Tego®Betain L7 (2,0% Tensidanteil) | Cocoamidopropyl Betaine |
| 0,30 Parfüm | |
| q.s. Konservierungsmittel | |
| Phase D | |
| q.s. Citronensäure | Citric Acid |
| 0,50 Natriumchlorid | Natriumchlorid |

Herstellung:

Die Komponenten der Phase A wurden eingewogen und gelöst, anschliessend mit NaOH ungefähr auf pH 6,7 eingestellt. Die Phase B wurde eingewogen und gelöst, anschliessend in Phase A gegeben. Die Komponenten der Phase C wurden in Phase A+B gegeben und homogen gerührt. Anschliessend wurde der pH-Wert mit Citronensäure auf ungefähr pH 6,0 eingestellt, Natriumchlorid zugegeben und das Gemisch homogen gerührt.

| Prüfdaten: | |
|---|---|
| Viskosität | 5340 mPas (Brookfield RVD VII+) |
| pH-Wert | 5,9 |

Anti-Schuppen-Shampoo mit Zink Pyrithione

| Ingredient | INCI (Hersteller) |
|---|---|
| Phase A | |
| 3,20 Dispersion 1 | |
| 41,00 Wasser dest. | Aqua dem. |
| q.s. NaOH 20% | Sodium Hydroxide |
| Phase B | |
| 0,80 Luviquat®Sensation (0,2% Polymeranteil) | Polyquaternium-87 |
| 2,90 Wasser dest. | Aqua dem. |
| Phase C | |
| 35,70 Texapon®NSO (9,7% Tensidanteil) | Sodium Laureth Sulfate |
| 12,50 Tego®Betain L7 (3,7% Tensidanteil) | Cocoamidopropyl Betaine |
| 2,50 Zink-Pyrion® 48% Micro (1,2% Aktivanteil) | Zink Pyrithione |
| Phase D | |
| 0,30 Parfüm | |
| q.s. Konservierungsmittel | |

(fortgesetzt)

| Ingredient | INCI (Hersteller) |
|---|---|
| Phase E | |
| q.s Citronensäure | Citronensäure |

Herstellung:

Die Komponenten der Phase A wurden eingewogen und gelöst, anschliessend mit NaOH ungefähr auf pH 6,7 eingestellt. Die Phase B wurde eingewogen und gelöst, anschliessend in Phase A gegeben. Die Komponenten der Phase C wurden in Phase A+B gegeben und homogen gerührt. Anschliessend wurde Phase D zugegeben und die erhaltene Mischung homogenisiert. Schließlich wurde der pH-Wert mit Citronensäure auf ungefähr pH 6,0 eingestellt.

| Prüfdaten: | |
|---|---|
| Viskosität | 12280 mPas (Brookfield RVD VII+) |
| pH-Wert | 6,2 |

Conditioning Shampoo mit Luviquat®Sensation, Uvinul®MC 80

| Ingredient | INCI (Hersteller) |
|---|---|
| Phase A | |
| 2,00 Luviquat®Sensation (0,5% Polymeranteil) | Polyquaternium-87 |
| 48,30 Wasser dest. | Aqua dem. |
| q.s. Konservierungsmittel | Preservative |
| Phase B | |
| 8,90 Dehyton®PK 45 | Cocamidopropyl Betaine |
| 35,70 Texapon®NSO (9,7% Tensidanteil) | Sodium Laureth Sulfate |
| Phase C | |
| 1,60 Dispersion 1 (0,5% Verdickeranteil) | |
| q.s. NaOH 20% | Sodium Hydroxide |
| Phase D | |
| 0,50 D-Panthenol®USP | Panthenol |
| 2,00 Uvinul®MC 80 | Ethylhexyl Methoxycinnamate |
| 0,30 Parfümöl | Perfume |
| 0,10 Edeta BD | Disodium EDTA |
| Phase E | |
| 0,70 Natriumchlorid | Sodium Chloride |

Herstellung:

Die Komponenten der Phase A wurden eingewogen und gemischt. Phase B wurde nacheinander in Phase A gegeben und gelöst. Die Komponenten der Phase C wurden der Phase A+B zugegeben, der pH-Wert auf ca. pH 6 eingestellt und bis zur Homogenität gerührt. Phase D wurde zugegeben und gelöst. Die Viskosität wurde mit Phase E eingestellt.

Prüfdaten:

(fortgesetzt)

| | |
|---|---|
| Viskosität | 4010 mPas (Brookfield RVD VII+) |
| pH-Wert | 5,91 |
| Reduktion der Nasskämmbarkeit | 70% ±5% |

Conditioning Shampoo mit Salcare®SC 60

| Ingredient | INCI (Hersteller) |
|---|---|
| Phase A | |
| 0,10 Salcare®SC 60 | Acrylamidepropyltrimonium Chloride, Acrylamide |
| 15,00 Wasser dest. | Aqua dem. |
| q.s. Konservierungsmittel | Preservative |
| Phase B | |
| 1,60 Dispersion 1 (0,5% Verdickeranteil) | |
| 33,30 Wasser dest. | Aqua dem. |
| q.s. NaOH 20% | Sodium Hydroxide |
| Phase C | |
| 8,90 Dehyton®PK 45 | Cocamidopropyl Betaine |
| 35,70 Texapon®NSO (9,7% Tensidanteil) | Sodium Laureth Sulfate |
| Phase D | |
| 0,50 D-Panthenol®USP | Panthenol |
| 4,00 Dow Corning®1664 | Dimethicone, Laureth-4, Laureth-23 |
| 0,30 Parfümöl | Perfume |
| Phase E | |
| 0,50 Natriumchlorid | Sodium Chloride |

Herstellung:

Die Komponenten der Phase A wurden eingewogen und gelöst. Phase B wurde eingewogen und mit NaOH neutralisiert. Phase C wurde in Phase B gegeben und homogen gerührt. Danach wurde die gelöste Phase A in Phase B+C gegeben und homogen gerührt. Der pH-Wert wurde auf ca. pH=6 eingestellt. Phase D wurde zugegeben und gelöst. Mit Phase E wurde die Viskosität eingestellt.

| | |
|---|---|
| Prüfdaten: | |
| Viskosität | 4920 mPas (Brookfield RVD VII+) |
| pH-Wert | 6,04 |
| Reduktion der Nasskämmbarkeit | 74% ± 4% |

Haarfarben-Formulierung - Entwickler

| Ingredient' | INCI (Hersteller) |
|---|---|
| Phase A | |

(fortgesetzt)

| Ingredient' | INCI (Hersteller) |
|---|---|
| 80,00 Wasser dest. | Aqua dem. |
| 0,15 Disodium Phosphate | Disodium Phosphate |
| | |
| Phase B | |
| 5,00 Dispersion 1 (1,6% Verdickeranteil) | |
| | |
| Phase C | |
| 12,00 Wasserstoffperoxid (50%) | Hydrogen Peroxid |
| 1,00 Glycerin (99%) | Glycerin |
| 1,00 Texapon®NSO (0,27% Tensidanteil) | Sodium Laureth Sulfate |
| | |
| Phase D | |
| q.s. Sequestrante HEDP | Etidronic Acid |

Herstellung:

Die Komponenten der Phase A wurden eingewogen und gelöst. Anschliessend wurden die Komponenten der Phasen B und C in Phase A gegeben und gerührt. Mit Phase D wurde der pH-Wert auf 2,5 bis 3 eingestellt.

## Patentansprüche

**1.** Verfahren zur Herstellung von Polymeren enthaltend in einpolymerisierter Form

a) M1a+M2a = 30 bis 70 Gew.-% eines Esters der (Meth)Acrylsäure mit einem $C_1$-$C_4$-Alkohol,
b) M1 b+M2b = 30 bis 70 Gew.-% (Meth)Acrylsäure,
c) M1c+M2c = 0,1 bis 20 Gew.-% mit wenigstens einem $C_8$-$C_{30}$-Rest substituierte Monomere,
d) M1d+M2d = 0 bis 20 Gew.-% von a) bis c) verschiedene Monomere, durch Emulsionspolymerisation mit einer ersten Polymerisationsstufe und einer zweiten Polymerisationsstufe, **dadurch gekennzeichnet, dass** M1b/M1a ≥ 0,4 und

M1b/M1a < M2b/M2a und $\sum_{i=a}^{d} M1i \leq 40$ Gew.-%,

wobei
M1 i die jeweiligen Gewichtsanteile der Monomere a) bis d) sind, die während der ersten Polymerisationsstufe im Reaktionsraum vorliegen und
M2i die jeweiligen Gewichtsanteile der Monomere a) bis d) sind, die während der zweiten Polymerisationsstufe zugegeben werden und die Summe aller Gewichtsanteile $\sum_{i=a}^{d} M1i + \sum_{i=a}^{d} M2i = 100$ Gew.-% ist.

**2.** Verfahren nach Anspruch 1, **dadurch gekennzeichnet, dass** a) Ethylacrylat umfasst.

**3.** Verfahren nach einem der Ansprüche 1 oder 2, **dadurch gekennzeichnet, dass** b) Methacrylsäure umfasst.

**4.** Verfahren nach einem der Ansprüche 1 bis 3, **dadurch gekennzeichnet, dass** c) 0,1 bis 20 Gew.-% eines Esters der (Meth)Acrylsäure mit wenigstens einem Alkohol der allgemeinen Formel (I)

$$H\text{-}(O\text{-}CHR\text{-}CH_2)_x\text{-}O\text{-}CyH_{2y+1} \qquad (I)$$

wobei x eine ganze Zahl von 10 bis 50 ist, y eine ganze Zahl von 12 bis 30 ist und R ausgewählt ist aus H und $CH_3$, in einpolymerisierter Form enthalten sind.

5. Verfahren nach Anspruch 4, **dadurch gekennzeichnet, dass** c) wenigstens zwei verschiedene Monomere der Formel (I) umfasst.

6. Verfahren nach einem der Ansprüche 4 oder 5, **dadurch gekennzeichnet, dass** y eine ganze Zahl von 16 bis 22 ist.

7. Verfahren nach einem der Ansprüche 1 bis 6, **dadurch gekennzeichnet, dass** die Emulsionspolymerisation bei pH <7 durchgeführt wird.

8. Verfahren nach einem der Ansprüche 1 bis 7, **dadurch gekennzeichnet, dass** die Emulsionspolymerisation bei pH <5 durchgeführt wird.

9. Verfahren nach einem der Ansprüche 1 bis 8, **dadurch gekennzeichnet, dass**

$$\sum_{i=a}^{d} M1i \leq 25 \text{ Gew.-\%.}$$

10. Verwendung eines Polymers erhältlich nach einem Verfahren nach einem der Ansprüche 1 bis 9 als Verdicker in wässrigen Zubereitungen.

11. Kosmetisches Mittel enthaltend wenigstens ein Polymer erhältlich nach einem Verfahren nach einem der Ansprüche 1 bis 9.

Abbildung 1: Versuchsaufbau Salzstabilität / schiefe Ebene

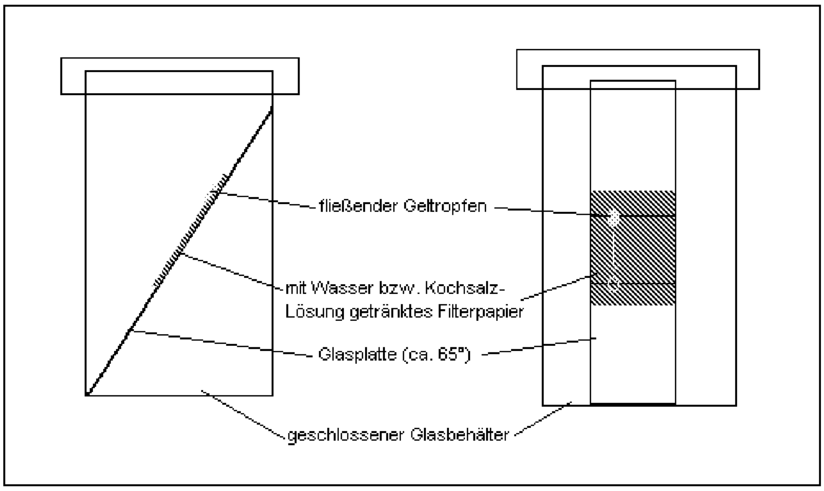

Europäisches Patentamt
European Patent Office
Office européen des brevets

# EUROPÄISCHER RECHERCHENBERICHT

Nummer der Anmeldung
EP 11 16 8919

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 5 436 292 A (JENKINS RICHARD D [US] ET AL) 25. Juli 1995 (1995-07-25)<br>* Spalten 1,2; Beispiel 63 *<br>----- | 1-11 | INV.<br>C08F220/06<br>C08F220/18<br>A61K8/81<br>C08F265/06<br>C08F2/00 |
| A,D | WO 2009/062994 A1 (BASF SE [DE]; LEYRER REINHOLD J [DE]; SCHMIDT KATI [DE]) 22. Mai 2009 (2009-05-22)<br>* Seiten 1,2; Beispiel 1 *<br>----- | 1-11 | |
| A,D | WO 99/65958 A1 (UNION CARBIDE CHEM PLASTIC [US]; BASSETT DAVID ROBINSON [US]; OLESEN K) 23. Dezember 1999 (1999-12-23)<br>* Seite 1, Absatz 1; Beispiel 1 *<br>----- | 1-11 | |
| | | | RECHERCHIERTE SACHGEBIETE (IPC) |
| | | | C08F<br>A61K |

Der vorliegende Recherchenbericht wurde für alle Patentansprüche erstellt

| Recherchenort | Abschlußdatum der Recherche | Prüfer |
|---|---|---|
| Den Haag | 7. September 2011 | Plehiers, Mark |

2

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.** EP 11 16 8919

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2011

| Im Recherchenbericht angeführtes Patentdokument | Datum der Veröffentlichung | Mitglied(er) der Patentfamilie | Datum der Veröffentlichung |
|---|---|---|---|
| US 5436292 A | 25-07-1995 | AT 154812 T | 15-07-1997 |
| | | AU 702693 B2 | 04-03-1999 |
| | | AU 7315294 A | 17-01-1995 |
| | | CA 2166195 A1 | 05-01-1995 |
| | | DE 69403967 D1 | 31-07-1997 |
| | | DE 69403967 T2 | 20-11-1997 |
| | | EP 0706535 A1 | 17-04-1996 |
| | | ES 2105737 T3 | 16-10-1997 |
| | | FI 956274 A | 27-02-1996 |
| | | JP 8512064 T | 17-12-1996 |
| | | NO 955315 A | 20-02-1996 |
| | | PL 307744 A1 | 12-06-1995 |
| | | US 5399618 A | 21-03-1995 |
| | | WO 9500565 A1 | 05-01-1995 |
| WO 2009062994 A1 | 22-05-2009 | AT 515519 T | 15-07-2011 |
| | | AU 2008322964 A1 | 22-05-2009 |
| | | CA 2703644 A1 | 22-05-2009 |
| | | CN 101861340 A | 13-10-2010 |
| | | EP 2209823 A1 | 28-07-2010 |
| | | JP 2011503312 A | 27-01-2011 |
| | | KR 20100113482 A | 21-10-2010 |
| | | US 2010210771 A1 | 19-08-2010 |
| WO 9965958 A1 | 23-12-1999 | AT 286080 T | 15-01-2005 |
| | | AU 4682899 A | 05-01-2000 |
| | | BR 9911170 A | 03-04-2001 |
| | | CA 2335242 A1 | 23-12-1999 |
| | | CN 1312828 A | 12-09-2001 |
| | | DE 69922962 D1 | 03-02-2005 |
| | | DE 69922962 T2 | 05-01-2006 |
| | | EP 1093478 A1 | 25-04-2001 |
| | | JP 2002518530 A | 25-06-2002 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 13836 A
- WO 9965958 A
- WO 2006016035 A
- WO 2009062994 A
- EP 761780 A2
- WO 2007010035 A
- WO 2006106140 A
- WO 2006106114 A

### In der Beschreibung aufgeführte Nicht-Patentliteratur

- Kosmetik und Hygiene von Kopf bis Fuß. Wiley-VCH, 2004, 235-236
- Kosmetik und Hygiene von Kopf bis Fuß. Wiley-VCH, 2004, 123-128